(19) Europäisches Patentamt
European Patent Office
Office européen des brevets

(11) **EP 1 237 001 A1**

(12) **EUROPEAN PATENT APPLICATION**
published in accordance with Art. 158(3) EPC

(43) Date of publication:
**04.09.2002 Bulletin 2002/36**

(21) Application number: **00977973.7**

(22) Date of filing: **28.11.2000**

(51) Int Cl.[7]: **G01N 33/50**, G01N 33/15, A61K 45/00

(86) International application number:
**PCT/JP00/08363**

(87) International publication number:
**WO 01/040797 (07.06.2001 Gazette 2001/23)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **29.11.1999 JP 33838799**
**24.02.2000 JP 2000052251**

(71) Applicant: **Takeda Chemical Industries, Ltd.**
**Osaka-shi, Osaka 541-0045 (JP)**

(72) Inventors:
- **HINUMA, Shuji**
**Tsukuba-shi, Ibaraki 305-0821 (JP)**
- **HOSOYA, Masaki**
**Tsuchiura-shi, Ibaraki 300-007 (JP)**
- **KAWAMATA, Yuji**
**Tsukuba-shi, Ibaraki 305-0035 (JP)**
- **FUKUSUMI, Shoji**
**Tsukuba-shi, Ibaraki 305-0044 (JP)**

(74) Representative: **Lewin, John Harvey**
**Takeda Patent Office,**
**11-12 Charles II Street**
**London SW1Y 4QU (GB)**

(54) **SCREENING METHOD**

(57) A method of screening a compound or a salt thereof that alters the binding property of Neuromedin U with FM-3, characterized by using Neuromedin U, a derivative thereof or a salt thereof and FM-3 or a salt thereof of the present invention, can be used for screening a therapeutic and/or prophylactic agent for hypertension or stress-related diseases. A FM-3 antagonist is useful as a therapeutic and/or prophylactic agent for hypertension or stress-related diseases.

EP 1 237 001 A1

[Sequence Listing]

<110> Takeda Chemical Industries, Ltd.

<120> Screening Method

<130> 2683WOOP

<150> JP 11-338387

<151> 1999-11-29

<150> JP 12-052251

<151> 2000-02-24

<160> 22

<210> 1

<211> 403

<212> PRT

<213> Human

<400> 1

Met Ala Cys Asn Gly Ser Ala Ala Arg Gly His Phe Asp Pro Glu Asp

1 5 10 15

Leu Asn Leu Thr Asp Glu Ala Leu Arg Leu Lys Tyr Leu Gly Pro Gln

20 25 30

Gln Thr Glu Leu Phe Met Pro Ile Cys Ala Thr Tyr Leu Leu Ile Phe

35 40 45

Val Val Gly Ala Val Gly Asn Gly Leu Thr Cys Leu Val Ile Leu Arg

50 55 60

His Lys Ala Met Arg Thr Pro Thr Asn Tyr Tyr Leu Phe Ser Leu Ala

65 70 75 80

Val Ser Asp Leu Leu Val Leu Leu Val Gly Leu Pro Leu Glu Leu Tyr

85 90 95

Glu Met Trp His Asn Tyr Pro Phe Leu Leu Gly Val Gly Gly Cys Tyr
100 105 110
Phe Arg Thr Leu Leu Phe Glu Met Val Cys Leu Ala Ser Val Leu Asn
115 120 125
Val Thr Ala Leu Ser Val Glu Arg Tyr Val Ala Val Val His Pro Leu
130 135 140
Gln Ala Arg Ser Met Val Thr Arg Ala His Val Arg Arg Val Leu Gly
145 150 155 160
Ala Val Trp Gly Leu Ala Met Leu Cys Ser Leu Pro Asn Thr Ser Leu
165 170 175
His Gly Ile Arg Gln Leu His Val Pro Cys Arg Gly Pro Val Pro Asp
180 185 190
Ser Ala Val Cys Met Leu Val Arg Pro Arg Ala Leu Tyr Asn Met Val
195 200 205
Val Gln Thr Thr Ala Leu Leu Phe Phe Cys Leu Pro Met Ala Ile Met
210 215 220
Ser Val Leu Tyr Leu Leu Ile Gly Leu Arg Leu Arg Arg Glu Arg Leu
225 230 235 240
Leu Leu Met Gln Glu Ala Lys Gly Arg Gly Ser Ala Ala Ala Arg Ser
245 250 255
Arg Tyr Thr Cys Arg Leu Gln Gln His Asp Arg Gly Arg Arg Gln Val
260 265 270
Thr Lys Met Leu Phe Val Leu Val Val Val Phe Gly Ile Cys Trp Ala
275 280 285
Pro Phe His Ala Asp Arg Val Met Trp Ser Val Val Ser Gln Trp Thr
290 295 300

Asp Gly Leu His Leu Ala Phe Gln His Val His Val Ile Ser Gly Ile
305 310 315 320
Phe Phe Tyr Leu Gly Ser Ala Ala Asn Pro Val Leu Tyr Ser Leu Met
325 330 335
Ser Ser Arg Phe Arg Glu Thr Phe Gln Glu Ala Leu Cys Leu Gly Ala
340 345 350
Cys Cys His Arg Leu Arg Pro Arg His Ser Ser His Ser Leu Ser Arg
355 360 365
Met Thr Thr Gly Ser Thr Leu Cys Asp Val Gly Ser Leu Gly Ser Trp
370 375 380
Val His Pro Leu Ala Gly Asn Asp Gly Pro Glu Ala Gln Gln Glu Thr
385 390 395 400
Asp Pro Ser
403

<210> 2
<211> 1209
<212> DNA
<213> Human
<400> 2

ATGGCTTGCA ATGGCAGTGC GGCCAGGGGG CACTTTGACC CTGAGGACTT GAACCTGACT 60
GACGAGGCAC TGAGACTCAA GTACCTGGGG CCCCAGCAGA CAGAGCTGTT CATGCCCATC 120
TGTGCCACAT ACCTGCTGAT CTTCGTGGTG GGCGCTGTGG GCAATGGGCT GACCTGTCTG 180
GTCATCCTGC GCCACAAGGC CATGCGCACG CCTACCAACT ACTACCTCTT CAGCCTGGCC 240
GTGTCGGACC TGCTGGTGCT GCTGGTGGGC CTGCCCCTGG AGCTCTATGA GATGTGGCAC 300
AACTACCCCT TCCTGCTGGG CGTTGGTGGC TGCTATTTCC GCACGCTACT GTTTGAGATG 360
GTCTGCCTGG CCTCAGTGCT CAACGTCACT GCCCTGAGCG TGGAACGCTA TGTGGCCGTG 420

GTGCACCCAC TCCAGGCCAG GTCCATGGTG ACGCGGGCCC ATGTGCGCCG AGTGCTTGGG 480

GCCGTCTGGG GTCTTGCCAT GCTCTGCTCC CTGCCCAACA CCAGCCTGCA CGGCATCCGG 540

CAGCTGCACG TGCCCTGCCG GGGCCCAGTG CCAGACTCAG CTGTTTGCAT GCTGGTCCGC 600

CCACGGGCCC TCTACAACAT GGTAGTGCAG ACCACCGCGC TGCTCTTCTT CTGCCTGCCC 660

ATGGCCATCA TGAGCGTGCT CTACCTGCTC ATTGGGCTGC GACTGCGGCG GGAGAGGCTG 720

CTGCTCATGC AGGAGGCCAA GGGCAGGGGC TCTGCAGCAG CCAGGTCCAG ATACACCTGC 780

AGGCTCCAGC AGCACGATCG GGGCCGGAGA CAAGTGACCA AGATGCTGTT TGTCCTGGTC 840

GTGGTGTTTG GCATCTGCTG GGCCCCGTTC CACGCCGACC GCGTCATGTG GAGCGTCGTG 900

TCACAGTGGA CAGATGGCCT GCACCTGGCC TTCCAGCACG TGCACGTCAT CTCCGGCATC 960

TTCTTCTACC TGGGCTCGGC GGCCAACCCC GTGCTCTATA GCCTCATGTC CAGCCGCTTC 1020

CGAGAGACCT TCCAGGAGGC CCTGTGCCTC GGGGCCTGCT GCCATCGCCT CAGACCCCGC 1080

CACAGCTCCC ACAGCCTCAG CAGGATGACC ACAGGCAGCA CCCTGTGTGA TGTGGGCTCC 1140

CTGGGCAGCT GGGTCCACCC CCTGGCTGGG AACGATGGCC CAGAGGCGCA GCAAGAGACC 1200

GATCCATCC

1209

<210> 3

<211> 405

<212> PRT

<213> Mouse

<400> 3

Met Val Cys Asn Ile Ser Glu Phe Lys Trp Pro Tyr Gln Pro Glu Asp

5 10 15

Leu Asn Leu Thr Asp Glu Ala Leu Arg Leu Lys Tyr Leu Gly Pro Gln

20 25 30

Gln Met Lys Gln Phe Val Pro Ile Cys Val Thr Tyr Leu Leu Ile Phe

35 40 45

Val Val Gly Thr Leu Gly Asn Gly Leu Thr Cys Thr Val Ile Leu Arg
50 55 60
Asn Lys Thr Met Arg Thr Pro Thr Asn Phe Tyr Leu Phe Ser Leu Ala
65 70 75 80
Val Ser Asp Met Leu Val Leu Leu Val Gly Leu Pro Leu Glu Leu Tyr
85 90 95
Glu Met Gln Gln Asn Tyr Pro Phe Gln Leu Gly Ala Ser Ala Cys Tyr
100 105 110
Phe Arg Ile Leu Leu Leu Glu Thr Val Cys Leu Ala Ser Val Leu Asn
115 120 125
Val Thr Ala Leu Ser Val Glu Arg Tyr Val Ala Val Val Arg Pro Leu
130 135 140
Gln Ala Lys Ser Val Met Thr Arg Ala His Val Arg Arg Met Val Gly
145 150 155 160
Ala Ile Trp Val Leu Ala Thr Leu Phe Ser Leu Pro Asn Thr Ser Leu
165 170 175
His Gly Leu Ser Gln Leu Thr Val Pro Cys Arg Gly Pro Val Pro Asp
180 185 190
Ser Ala Ile Cys Ser Leu Val Gly Pro Met Asp Phe Tyr Lys Leu Val
195 200 205
Val Leu Thr Thr Ala Leu Leu Phe Phe Cys Leu Pro Met Val Thr Ile
210 215 220
Ser Val Leu Tyr Leu Leu Ile Gly Leu Arg Leu Arg Arg Glu Arg Met
225 230 235 240
Leu Leu Gln Val Glu Val Lys Gly Arg Lys Thr Ala Ala Thr Gln Glu
245 250 255

Thr Ser His Arg Arg Ile Gln Leu Gln Asp Arg Gly Arg Arg Gln Val

260 265 270

Thr Lys Met Leu Phe Ala Leu Val Val Val Phe Gly Ile Cys Trp Ala

275 280 285

Pro Phe His Ala Asp Arg Ile Met Trp Ser Leu Val Tyr Gly His Ser

290 295 300

Thr Glu Gly Leu His Leu Ala Tyr Gln Cys Val His Ile Ala Ser Gly

305 310 315 320

Ile Phe Phe Tyr Leu Gly Ser Ala Ala Asn Pro Val Leu Tyr Ser Leu

325 330 335

Met Ser Thr Arg Phe Arg Glu Thr Phe Leu Gln Ala Leu Gly Leu Gly

340 345 350

Thr Gln Cys Cys His Arg Arg Gln Pro Tyr His Gly Ser His Asn His

355 360 365

Ile Arg Leu Thr Thr Gly Ser Thr Leu Cys Asp Val Gly His Arg Asn

370 375 380

Ser Arg Asp Glu Pro Leu Ala Val Asn Glu Asp Pro Gly Cys Gln Gln

385 390 395 400

Glu Thr Asp Pro Ser

405

<210> 4

<211> 1215

<212> DNA

<213> Mouse

<400> 4

ATGGTCTGCA ATATCAGTGA GTTCAAGTGG CCCTATCAAC CTGAGGATCT GAACCTTACC 60

GATGAGGCCC TGAGGCTGAA GTATTTGGGG CCACAGCAGA TGAAACAGTT TGTCCCCATC 120

TGTGTCACGT ACCTGCTGAT CTTCGTGGTG GGCACTCTGG GCAACGGGCT GACCTGCACC 180

GTCATCCTGC GCAACAAGAC TATGCGCACG CCCACCAACT TCTACCTCTT CAGCCTCGCT 240

GTGTCCGATA TGCTGGTGCT CCTGGTGGGC TTGCCTCTGG AGCTTTATGA GATGCAGCAA 300

AATTACCCGT TCCAGCTGGG TGCGAGTGCC TGCTACTTCC GAATACTGCT CTTAGAGACC 360

GTCTGCCTAG CTTCAGTGCT CAATGTCACA GCCCTGAGTG TGGAGCGTTA TGTGGCCGTG 420

GTGCGCCCAC TCCAAGCCAA GTCTGTGATG ACACGGGCCC ATGTGCGCCG CATGGTGGGG 480

GCCATCTGGG TCCTCGCTAC TCTCTTCTCT CTGCCCAACA CCAGCCTGCA TGGCCTCAGT 540

CAACTAACTG TGCCCTGCCG GGGGCCGGTG CCCGACTCAG CTATATGTTC GCTGGTGGGT 600

CCCATGGACT TCTACAAGTT GGTGGTACTG ACTACCGCAC TGCTCTTCTT CTGTCTGCCC 660

ATGGTCACCA TCAGTGTGCT GTATCTGCTC ATTGGGCTGC GGCTGCGGAG GGAGAGGATG 720

TTGCTCCAAG TGGAGGTCAA GGGCAGGAAA ACCGCAGCAA CCCAGGAGAC CTCCCACAGA 780

AGGATTCAGC TGCAAGATAG GGGACGGAGA CAGGTGACCA AGATGCTGTT TGCACTGGTT 840

GTGGTATTCG GCATCTGCTG GGCTCCATTC CATGCTGACC GTATCATGTG GAGCCTGGTG 900

TATGGACACT CAACGGAAGG CCTGCACCTG GCCTACCAGT GTGTCCACAT TGCCTCTGGC 960

ATCTTCTTCT ATCTCGGCTC AGCAGCCAAC CCGGTGCTCT ACAGCCTCAT GTCTACTCGC 1020

TTCCGAGAGA CCTTCCTGCA AGCCCTGGGC CTTGGAACCC AGTGCTGTCA TCGCCGCCAA 1080

CCCTATCATG GCTCCCATAA CCACATCAGG TTGACCACAG GCAGCACCCT GTGTGACGTG 1140

GGCCACAGGA ACAGCAGGGA CGAACCTCTG GCTGTGAATG AGGATCCAGG GTGTCAGCAA 1200

GAGACAGACC CCTCC 1215

<210> 5

<211> 8

<212> PRT

<213> Pig

<400> 5

Tyr Phe Leu Phe Arg Pro Arg Asn

1 5 8

<210> 6

<211> 8

<212> PRT

<213> Dog

<400> 6

Glu Phe Leu Phe Arg Pro Arg Asn

1 5 8

<210> 7

<211> 9

<212> PRT

<213> Chicken

<400> 7

Gly Tyr Phe Phe Phe Arg Pro Arg Asn

1 5 9

<210> 8

<211> 9

<212> PRT

<213> Guinea pig

<400> 8

Gly Tyr Phe Leu Phe Arg Pro Arg Asn

1 5 9

<210> 9

<211> 23

<212> PRT

<213> Rat

<400> 9

Tyr Lys Val Asn Glu Tyr Gln Gly Pro Val Ala Pro Ser Gly Gly Phe

1 5 10 15

Phe Leu Phe Arg Pro Arg Asn

20 23

<210> 10

<211> 23

<212> PRT

<213> Frog

<400> 10

Ser Asp Glu Glu Val Gln Val Pro Gly Gly Val Ile Ser Asn Gly Tyr

1 5 10 15

Phe Leu Phe Arg Pro Arg Asn

20 23

<210> 11

<211> 25

<212> PRT

<213> Human

<400> 11

Phe Arg Val Asp Glu Glu Phe Gln Ser Pro Phe Ala Ser Gln Ser Arg

1 5 10 15

Gly Tyr Phe Leu Phe Arg Pro Arg Asn

20 25

<210> 12

<211> 25

<212> PRT

<213> Pig

<400> 12

Phe Leu Val Asp Glu Glu Phe Gln Gly Pro Ile Val Ser Gln Asn Arg

1 5 10 15

Arg Tyr Phe Leu Phe Arg Pro Arg Asn

20 25

<210> 13

<211> 25

<212> PRT

<213> Dog

<400> 13

Phe Arg Leu Asp Glu Glu Phe Gln Gly Pro Ile Ala Ser Gln Val Arg

1 5 10 15

Arg Gln Phe Leu Phe Arg Pro Arg Asn

20 25

<210> 14

<211> 25

<212> PRT

<213> Chicken

<400> 14

Tyr Lys Val Asp Glu Asp Leu Gln Gly Ala Gly Gly Ile Gln Ser Arg

1 5 10 15

Gly Tyr Phe Phe Phe Arg Pro Arg Asn

20 25

<210> 15

<211> 25

<212> PRT

<213> Frog

<400> 15

Leu Lys Pro Asp Glu Glu Leu Gln Gly Pro Gly Gly Val Leu Ser Arg

1 5 10 15

Gly Tyr Phe Val Phe Arg Pro Arg Asn

20 25

<210> 16

<211> 5

<212> PRT

<213> Human

<400> 16

Phe Arg Pro Arg Asn

1 5

<210> 17

<211> 33

<212> DNA

<213> Artificial Sequence

<220>

<223>

<400> 17

GTCGACCATG GCTTGCAATG GCAGTGCGGCC AGG 33

<210> 18

<211> 30

<212> DNA

<213> Artificial Sequence

<220>

<223>

<400> 18

GCTAGCTCAG GATGGATCGG TCTCTTGCTG 30

<210> 19

<211> 412

<212> PRT

<213> Rat

<400> 19

Met Leu Ser Pro Asn Ala Ser Thr Gly Leu Leu Ser Cys Asn Asp Ser
5 10 15

Glu Phe Lys Glu His Phe Asp Leu Glu Asp Leu Asn Leu Thr His Glu
20 25 30

Asp Leu Arg Leu Lys Tyr Leu Gly Pro Gln Gln Val Lys Gln Phe Leu
35 40 45

Pro Ile Cys Val Thr Tyr Leu Leu Ile Phe Val Val Gly Thr Leu Gly
50 55 60

Asn Gly Leu Thr Cys Thr Val Ile Leu Arg Gln Lys Ala Met His Thr
65 70 75 80

Pro Thr Asn Phe Tyr Leu Phe Ser Leu Ala Val Ser Asp Leu Leu Val
85 90 95

Leu Leu Val Gly Leu Pro Leu Glu Leu Tyr Glu Met Gln His Asn Tyr
100 105 110

Pro Phe Gln Leu Gly Ala Gly Gly Cys Tyr Phe Arg Ile Leu Leu Leu
115 120 125

Glu Thr Val Cys Leu Ala Ser Val Leu Asn Val Thr Ala Leu Ser Val

130 135 140

Glu Arg Tyr Val Ala Val Val His Pro Leu Gln Ala Lys Ser Val Met

145 150 155 160

Thr Arg Thr His Val Arg Arg Met Leu Gly Ala Ile Trp Val Phe Ala

165 170 175

Ile Leu Phe Ser Leu Pro Asn Thr Ser Leu His Gly Leu Ser Pro Leu

180 185 190

Tyr Val Pro Cys Arg Gly Pro Val Pro Asp Ser Val Thr Cys Thr Leu

195 200 205

Val Arg Pro Gln Phe Phe Tyr Lys Leu Val Ile Gln Thr Thr Ile Leu

210 215 220

Leu Phe Phe Cys Leu Pro Met Val Thr Ile Ser Val Leu Tyr Leu Leu

225 230 235 240

Ile Gly Leu Arg Leu Arg Arg Glu Arg Met Leu Leu Gln Glu Glu Val

245 250 255

Lys Gly Arg Ile Ser Ala Ala Ala Arg Gln Ala Ser His Arg Ser Ile

260 265 270

Gln Leu Arg Asp Arg Glu Arg Arg Gln Val Thr Lys Met Leu Ile Ala

275 280 285

Leu Val Ile Val Phe Gly Thr Cys Trp Val Pro Phe His Ala Asp Arg

290 295 300

Leu Met Trp Ser Met Val Ser His Trp Thr Asp Gly Leu Arg Leu Ala

305 310 315 320

Phe Gln Ser Val His Leu Ala Ser Gly Val Phe Leu Tyr Leu Gly Ser

325 330 335

Ala Ala Asn Pro Glu Leu Tyr Asn Leu Met Ser Thr Arg Phe Arg Glu

340 345 350

Ser Phe Arg Glu Thr Leu Gly Leu Gly Thr Arg Cys Cys His Arg His

355 360 365

Gln Pro Arg His Asp Ser His Ser His Leu Arg Leu Thr Thr Val Ser

370 375 380

Thr Leu Cys Asp Arg Asn Ser Arg Asp Val Pro Leu Ala Glu Asn Arg

385 390 395 400

Asp Pro Gly Cys Glu Gln Glu Thr Asp Pro Pro Glu

405 410 412

<210> 20

<211> 1239

<212> DNA

<213> Rat

<400> 20

```
ATGCTCTCCC CAAATGCTTC AACGGGCCTC TTGTCCTGCA ATGACAGTGA GTTCAAGGAG    60
CACTTTGACC TTGAGGACCT GAACCTTACT CATGAGGACC TGAGGCTGAA GTACTTGGGG   120
CCACAGCAGG TAAAACAATT TTTGCCCATC TGTGTCACGT ACCTGTTGAT CTTCGTAGTG   180
GGCACTCTGG GCAACGGGTT GACCTGCACC GTCATCCTGC GCCAGAAGGC AATGCACACG   240
CCCACCAACT TCTACCTCTT CAGTCTCGCG GTGTCCGATT TGCTGGTGCT CCTGGTGGGC   300
TTGCCCCTGG AACTTTATGA GATGCAGCAC AATTACCCAT TCCAGCTGGG TGCAGGTGGC   360
TGTTACTTCC GGATACTGCT TTTGGAGACT GTCTGCCTGG CTTCAGTGCT CAATGTCACA   420
GCCCTAAGTG TGGAGCGTTA TGTGGCCGTG GTGCACCCAC TCCAAGCCAA GTCTGTGATG   480
ACACGGACCC ATGTGCGCCG CATGTTGGGA GCCATCTGGG TCTTCGCTAT TCTCTTCTCT   540
CTGCCCAACA CCAGCTTACA TGGCCTCAGT CCACTCTATG TACCCTGCCG GGGGCCGGTG   600
CCCGATTCAG TTACGTGTAC GCTGGTGCGT CCCCAGTTCT TCTACAAGTT GGTAATACAG   660
ACGACCATAC TGCTCTTCTT CTGTCTGCCC ATGGTCACCA TCAGTGTGCT GTACCTGCTC   720
```

ATTGGGCTGA GGCTGCGGAG GGAGAGGATG TTGCTCCAAG AGGAGGTCAA GGGCAGGATA 780

TCTGCAGCAG CCAGGCAGGC CTCCCACAGA AGTATTCAGC TTCGAGATAG GGAACGCAGA 840

CAGGTGACCA AGATGCTAAT TGCTCTGGTT ATAGTATTTG GCACCTGCTG GGTTCCATTC 900

CATGCTGACC GTCTCATGTG GAGTATGGTG TCCCATTGGA CTGACGGCCT GCGCCTGGCC 960

TTCCAGTCTG TGCACCTTGC TTCTGGTGTC TTCTTGTACC TCGGCTCAGC GGCTAACCCG 1020

GAGCTCTACA ACCTCATGTC CACTCGCTTC CGAGAGTCCT TCCGGGAAAC CCTGGGCCTT 1080

GGGACACGGT GCTGTCATCG CCACCAACCG CGTCACGACT CCCATAGCCA CCTTAGGTTG 1140

ACCACAGTCA GCACCCTGTG TGACAGGAAC AGCAGGGATG TACCCCTGGC TGAGAACAGG 1200

GATCCAGGGT GTGAGCAAGA GACAGACCCT CCTGAATAA 1239

<210> 21

<211> 33

<212> DNA

<213> Artificial Sequence

<220>

<223>

<400> 21

GTCGACCATG CTCTCCCCAA ATGCTTCAAC GGG 33

<210> 22

<211> 33

<212> DNA

<213> Artificial Sequence

<220>

<223>

<400> 22

GCTAGCTTAT TCAGGAGGGT CTGTCTCTTG CTC 33

## Description

FIELD OF THE INVENTION

The present invention relates to a screening method for a prophylactic and/or therapeutic agent for hypertension or stress-related diseases, or an agent for controlling appetite, which is characterized by using an orphan receptor FM-3 (Tan, C.P. et al., Genomics 52, 223-229, 1998, etc) or a salt thereof, and Neuromedin U (Minamino, N. et al., Biochem. Biophys. Res. Commmun. 130, 1078-1085, 1985), a derivative thereof or a salt thereof.

BACKGROUND ART

A variety of hormones and neurotransmitters regulate the biological functions through specific receptor proteins located in a cell membrane. Many of these receptor proteins are coupled with guanine nucleotide-binding proteins (hereinafter sometimes referred to as G proteins) and evoke the intracellular signal transduction via activation of the G proteins. These receptor proteins possess the common structure, i.e. seven transmembrane domains and are thus collectively referred to as G protein-coupled receptors or seven-transmembrane receptors (7TMR).

An important regulation of biological functions, such as homeostasis, reproduction, individual development, metabolism, growth, regulations of nervous system, respiratory system, digestive system and metabolic system, and sensory system is conducted through an interaction between these hormones or neurotransmitters and G protein-coupled receptor proteins. In this context, it is known that there are various receptor proteins for hormones and neurotransmitters for the regulation of biological functions and these proteins play an important role for regulating the functions. However, it is not much clear as to whether unknown active substances (e.g. hormones, neurotransmitters, etc.) and receptors thereof still exist.

In recent years, using the fact that G protein-coupled receptor proteins represent similarities in their partial amino acid sequences, the search for DNA encoding a novel receptor protein is conducted by Polymerase Chain Reaction (hereinafter abbreviated as PCR) method. Therefore, many orphan G protein-coupled receptor proteins whose ligand are not known, are cloned (Libert, F., et al. Science, 244, 569-572, 1989, Welch, S.K., et al., Biochem. Biophys. Res. Commun., 209, 609-613, 1995, Marchese, A., et al., Genomics, 23, 609-618, 1994, Marchese, A., Genomics, 29, 335-344, 1995). Novel G protein-coupled receptor proteins are also found by random analysis of genomic DNA or cDNA sequences(Nomura, N., et al., DNA Research vol. 1, 27-25,1994). General methods for determining a ligand to an orphan G protein-coupled receptor protein are only to predict the ligand from similarity of the primary structure of G protein-coupled receptor protein. However, since many G protein-coupled receptor proteins represent low homology with the known receptors, it is difficult to predict a ligand only from the similarity of the primary structure unless the receptor protein is a subtype receptor for the known ligand. On the other hand, many orphan G protein-coupled receptor proteins are found by genetic analysis. So, it is estimated that there are many unknown ligands still remained. Nevertheless, only a few ligands for G protein-coupled receptor proteins are actually identified.

FM-3 is a seven-transmembrane receptor (7TMR), obtained as a receptor that is similar to the receptor for growth hormone secretagogues (GHS-R) and Neurotensin receptor(NT-R). Identity of amino acid sequence of FM-3 is 33% with GHS-R and 29% with NT-R type 1 (Tan, C.P. et al., Genomics 52, 223-229, 1998). The receptors similar to GHS-R include GPR38, GPR39, etc. (McKee, K.K. et al., Genomics 46, 426-434, 1997). These receptors are considered to form a receptor family as a whole.

Generally, among structurally similar receptors, their ligands show cross reaction to each other. However, it is reported that labeled MK-0677 (GHS type) and Neurotensin did not bind with HEK-239 cells which were confirmed to express FM-3 on the cell membrane after introduction of FM-3 gene(Tan, C.P. et al., Genomics 52, 223-229, 1998). Moreover, peptides such as endothelin, VIP (vasoactive intestinal peptide), gastorins, growth hormone releasing hormone (GH-RH), somatostatin, thyrotropin releasing hormone (TRH), calcitonin, galanin are also examined for activation of FM-3. However, the activation of FM-3 is not detected in an assay where free intracellular calcium ion release is measured as an index (Tan, C.P. et al., Biochem. Biophys. Res. Commun. 130, 1078-1085,1985). Thus, the information about endogenous ligands for FM-3 has not yet been obtained until now.

On the other hand, Neuromedin U is a peptide, which was isolated and purified from porcine spinal cords, with setting a rat uterus smooth muscle contraction activity as an index. Two kinds of Neuromedin U, Neuromedin U-8 having 8 amino acid residues and Neuromedin U-25 having 25 amino acid residues are first reported (Minamino, N. et al., Biochem. Biophys. Res. Commun. 130, 1078-1085, 1985). Since the sequence of Neuromedin U-8 is identical to C-terminal sequence of Neuromedin U-25 and the upstream region contains a basic amino acid pair often seen in the cleavage site for processing, both Neuromedin U are expected to be derived from a common precursor. Also, other physiological functions besides the smooth muscle contraction activity are widely known. Such functions reportedly include, for example, increase in blood pressure (Minamino. N. et al.), decrease in bloodstream of intestine (Sumi, S. et al., Life Sci. 41, 1585-1590, 1987), adjustment of ion transportation in intestine (Brown, D.R. and Quito, F.L., Eur.

J.Pharmacol. 155, 159-162, 1998) and increase in ACTH and subsequent increase in corticosterone after hypodermic administration of Neuromedin U. Generally, since physiologically active peptides work through the specific receptors, it is important to elucidate the properties and distributions of receptors to clarify the action mechanism of the peptide. However, the receptor of Neuromedin U has not been known.

SUMMARY OF THE INVENTION

The ligand for FM-3 has been unknown. However, the endogenous ligand, agonist and antagonist of FM-3 are needed to elucidate the physiological functions via FM-3 and to develop drugs. Furthermore, the receptor for Neuromedin U has also been unknown until now. It is necessary to clarify the structure of a Neuromedin U receptor in order to clarify the physiological role of Neuromedin U and to develop new drugs by screening the compound that activates, modifies and inhibits its mechanism.

The present inventors found with further extensive investigation that Neuromedin U has the cell stimulating activity to the FM-3-expressing CHO cells. Furthermore, cDNA of rat novel FM-3 was isolated and its complete sequence was successfully determined. Based on these findings, the present inventors continued extensive studies to accomplish the present invention.

Thus, the present invention relates to the followings:

(1) A method of screening a compound or a salt thereof that alters the binding property of Neuromedin U or a salt thereof with FM-3 or a salt thereof, which is characterized by using Neuromedin U, a derivative thereof or a salt thereof and FM-3 or a salt thereof.

(2) A kit for screening a compound a salt thereof that alters the binding property of Neuromedin U or a salt thereof with FM-3 or a salt thereof, which is characterized by comprising Neuromedin U, a derivative thereof or a salt thereof and FM-3 or a salt thereof.

(3) A compound or a salt thereof that alters the binding property of Neuromedin U or a salt thereof with FM-3 or a salt thereof, which is obtainable using the screening method described in (1) or the screening kit described in (2).

(4) A pharmaceutical composition comprising the compound described in (3).

(5) A pharmaceutical composition described in (4), which is a therapeutic and prophylactic agent for hypertension or stress-related diseases.

(6) An agent for controlling appetite, comprising the compound described in (3).

(7) A screening method described in (1) or a screening kit described in (2), where Neuromedin U is a peptide comprising the same or the substantially same amino acid sequence as that represented by SEQ ID NO:11.

(8) A screening method described in (1) or a screening kit described in (2), where FM-3 is a protein comprising the same or the substantially same amino acid sequence as that represented by SEQ ID NO:1.

(9) A protein or a salt thereof comprising (i) the amino acid sequence represented by SEQ ID NO:19, (ii) the amino acid sequence represented by SEQ ID NO:19, to which 1 to 30 amino acids are added, (iii) the amino acid sequence represented by SEQ ID NO:19, in which 1 to 30 amino acids are substituted with other amino acids, (iv) the amino acid sequence represented by SEQ ID NO:19, to which 1 to 30 amino acids are added and in which 1 to 30 amino acids are substituted with other amino acids.

(10) A DNA containing the DNA encoding the protein described in (9).

(11) A DNA described in (10) having the sequence represented by SEQ ID NO:20.

(12) A recombinant vector containing the DNA described in (10).

(13) A transformant transformed with the recombinant vector described in (12).

(14) A method for manufacturing the protein or a salt thereof described in (9), characterized by cultivating the transformant described in (13) and making the transformant produce the protein described in (9).

(15) An antibody to the protein or a salt thereof described in (9).

Regarding FM-3 of the present invention, concretely, in addition to the above-mentioned FM-3 or a salt thereof, the following are also listed.

(16) FM-3 or a salt thereof which is the protein containing the same or the substantially same amino acid sequence as than represented by SEQ ID NO:3 or SEQ ID NO:19.

(17) FM-3 or a salt thereof which is the protein containing: the amino acid sequence represented by SEQ ID NO:1, SEQ ID NO:3 or SEQ ID NO:19, wherein 1 to 30 amino acids, preferably 1 to 10 amino acids are deleted; the amino acid sequence represented by SEQ ID NO:1, SEQ ID NO:3 or SEQ ID NO:19, to which 1 to 30 amino acids, preferably 1 to 10 amino acids are added (inserted); or the amino acid sequence represented by SEQ ID NO:1, SEQ ID NO:3 or SEQ ID NO:19, in which 1 to 30 amino acids, preferably 1 to 10 amino acids are substituted with other amino acids.

Regarding Neuromedin U of the present invention, concretely, in addition to the above-mentioned Neuromedin U or a salt thereof, the following are also listed.

(18) Neuromedin U, a derivative or a salt thereof, which is the peptide containing the amino acid sequence represented by SEQ ID NO:5, SEQ ID NO:6, SEQ ID NO:7, SEQ ID NO:8, SEQ ID NO:9, SEQ ID NO:10, SEQ ID NO: 12, SEQ ID NO:13, SEQ ID NO:14 or SEQ ID NO:15.

In the present invention, preferred is Neuromedin U wherein the carboxyl group of the C-terminal amino acid is amidated.

The rat FM-3 of the present invention includes the protein or a salt thereof containing (i) the amino acid sequence represented by SEQ ID NO:19, (ii) the amino acid sequence represented by SEQ ID NO:19, to which 1 to 30 amino acids, preferably 1 to 10 amino acids are added, (iii) the amino acid sequence represented by SEQ ID NO: 19, in which 1 to 30 amino acids, preferably 1 to 10 amino acids are substituted with other amino acids, (iv) the amino acid sequence represented by SEQ ID NO:19, to which 1 to 30 amino acids, preferably 1 to 10 amino acids are added and in which 1 to 30 amino acids, preferably 1 to 10 amino acids are substituted with other amino acids.

BRIEF DESCRIPTION OF THE DRAWINGS

FIG. 1 shows the result of detection of the FM-3 specific cell-stimulating activity by site sensor, conducted in EXAMPLE 2. Pig Neuromedin U-8 at the concentration indicated in FIG. 1 was added to FM-3-expressing CHO cells (●) and mock CHO cells (○) for 7 minutes and 2 seconds in the site sensor assay. The maximum value of acidification rate in the reaction was plotted.

BEST MODE OF THE INVENTION

The details of method for producing FM-3 (including the above-mentioned rat FM-3) or a salt thereof (hereinafter simply referred to as FM-3) and Neuromedin U, a derivative thereof or a salt thereof (hereinafter simply referred to as Neuromedin U) are described as follows.

FM-3 and Neuromedin U of the present invention may be any proteins ((poly)peptides) derived from any tissues (e.g. hypophysis, pancreas, brain, kidney, liver, gonad, thyroid, gallbladder, bone marrow, adrenal gland, skin, muscle, lung, gastrointestinal tract, blood vessel, heart, etc.) or any cells from warm-blooded animals (e.g. human, guinea pig, rat, mouse, swine, sheep, bovine, monkey, dog, chicken), amphibian (e.g. frog) and fish. FM-3 may be any proteins ((poly)peptides) having the same or substantially the same amino acid sequence as that represented by SEQ ID NO:1, and Neuromedin U may be any proteins having the same or substantially the same amino acid sequence as that represented by SEQ ID NO:11.

For example, FM-3 of the present invention includes the protein having the substantially same activity as that of the protein having the substantially same amino acid sequence as that represented by SEQ ID NO:1, besides the protein containing the amino acid sequence represented by SEQ ID NO:1.

Herein, the term "substantially same" is intended to mean that a binding activity of a ligand (Neuromedin U) and a receptor (FM-3), a physiological property or the like is equivalent. The substitution, deletion, addition and insertion of amino acid often do not make a detectable change in physiological and chemical properties of polypeptide. In such case, the protein ((poly) peptide) that is modified by substitution, deletion, addition or insertion (what is called Neuromedin U variant, FM-3 variant) is considered to be substantially the same as the proteins which are not substituted, deleted, added and inserted.

The substantially same substituent as the amino acid in said amino acid sequence can be selected from, for example, any other amino acids of the group that the amino acid belongs to. Non-polar (hydrophobic) amino acid includes alanine, leucine, isoleucine, valine, proline, phenylalanine, tryptophan, methionine, etc. Polar (neutral) amino acid includes glycine, serine, threonin, cycteine, thyrosine, asparagine, glutamine, etc. Positively charged (basic) amino acid includes arginine, lysine, histidine, etc. Negatively charged (acidic) amino acid includes aspartic acid, glutamic acid, etc.

Therefore, the proteins may vary in quantitative factors such as a binding activity level, a molecular weight, etc. Examples of the protein having the substantially same activity as that of the protein having the amino acid sequence represented by SEQ ID NO:1 include a protein having the amino acid sequence represented by SEQ ID NO:3 and a protein having the amino acid sequence represented by SEQ ID NO:19, etc.

On the other hand, Neuromedin U of the present invention include the (poly) peptide having the substantially same activity as the (poly) peptide having the same amino acid sequence as that represented by SEQ ID NO:11, besides the (poly) peptide having the amino acid sequence represented by SEQ ID NO:11.

Examples of the substantially same activity include, for example, a ligand binding activity, etc. The term "substantially same" is intended to mean that binding activities of the receptors are the same in terms of property. Therefore, the proteins may vary in quantitative factors such as a binding activity level, a molecular weight, etc.

The (poly)peptides which contain the substantially same amino acid sequence as that represented by SEQ ID NO:11 include, for example, the polypeptide having the amino acid sequence represented by SEQ ID NO:5, the polypeptide having the amino acid sequence represented by SEQ ID NO:6, the polypeptide having the amino acid sequence represented by SEQ ID NO:7, the polypeptide having the amino acid sequence represented by SEQ ID NO: 8, the polypeptide having the amino acid sequence represented by SEQ ID NO:9, the polypeptide having the amino acid sequence represented by SEQ ID NO:10, the polypeptide having the amino acid sequence represented by SEQ ID NO:11, the polypeptide having the amino acid sequence represented by SEQ ID NO:12, the polypeptide having the amino acid sequence represented by SEQ ID NO:13, the polypeptide having the amino acid sequence represented by SEQ ID NO:14 and the polypeptide having the amino acid sequence represented by SEQ ID NO:15, etc.

In the present specification, the amino acid sequences of FM-3 and Neuromedin U are shown so that the N-terminal (amino terminal) is placed in the left and the C-terminal (carboxyl terminal) in the right, in accordance with a conventional peptide notation system. The protein or (poly) peptide having the amino acid sequence represented by SEQ ID NO: 1 or SEQ ID NO: 11 usually has a carboxyl group (-COOH) or carboxylate (-COO') at the C-terminal, but may have an amide ($-CONH_2$) or ester (-COOR) at the C-terminal.

R in such an ester includes, for example, $C_{1-6}$ alkyl groups such as methyl, ethyl, n-propyl, isopropyl and n-butyl; $C_{3-8}$ cycloalkyl groups such as cyclopentyl and cyclohexyl; $C_{6-12}$ aryl groups such as phenyl and α-naphthyl; $C_{7-14}$ aralkyl groups such as phenyl-$C_{1-2}$ alkyl, such as benzyl, phenethyl and benzhydryl, and α-naphthyl-$C_{1-2}$ alkyl, such as α-naphthylmethyl; and pivaloyloxymethyl groups generally used as an ester suitable for oral administration.

Preferred example of Neuromedin U of the present invention is the one having an amide ($-CONH_2$) at the C-terminal.

Examples of salts of FM-3 or Neuromedin U used in the present invention include salts with physiologically acceptable bases (e.g., alkali metal salts) or acids (e.g., inorganic acids, organic acids). Specially, physiologically acceptable acid addition salts are preferred. Such salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The FM-3 or Neuromedin U used in the present invention can be produced by per se known method (FEBS Letters, 398(1996), 253-258, the method used in WO 96/18651). That is, it can be produced by a polypeptide purification technique from the human or warm-blooded animal cells or tissues or can be produced by a peptide synthetic method described below as well. Alternatively, it can be produced by culturing a transformant containing the DNA encoding the protein described below.

Where the proteins are manufactured from tissues or cells of human, warm-blooded animal, amphibian or fish, the tissues or cells are homogenized, then extracted with an acid, an organic solvent or the like, and the proteins are isolated and purified from the extract by a combination of chromatography techniques such as salting out, dialysis, gel filtration, reverse-phase chromatography, ion-exchange chromatography, affinity chromatography, etc.

FM-3 or Neuromedin U used in the present invention can be produced according to a peptide synthesis method known per se or by cleaving the protein ((poly) peptide) having FM-3 or Neuromedin U with a suitable peptidase. For example, the protein ((poly) peptide) synthesis method may be the solid- or liquid-phase synthesis method. That is, the desired protein ((poly) peptide) can be obtained by condensation of a partial peptide or amino acids capable of constituting the FM-3 or Neuromedin U with the remainder, followed by elimination of protecting groups if any from the product. The known condensation method and the elimination of the protecting groups include the methods described in e.g. (1) to (5) below:

(1) M. Bodanszky and M. A. Ondetti, Peptide Synthesis, Interscience Publisher, New York (1966);
(2) Schroeder and Luebke, The Peptide, Academic Press, New York (1965);
(3) Nobuo Izumiya et al., Basis and Experiments in Peptide Synthesis, Maruzen Co., Ltd. (1975);
(4) Haruaki Yajima and Shunpei Sakakibara, Biochemical Experimental Course 1, Protein Chemistry IV, 205, (1977); and
(5) Haruaki Yajima (supervisor), Development of medicines, a second series, vol.14, Peptide Synthesis, Hirokawashoten.

After the reaction, the protein ((poly) peptide) can be isolated and purified by a combination of conventional purification techniques such as solvent extraction, distillation, column chromatography, liquid chromatography and recrystallization. If the protein ((poly) peptide) is obtained in a free form by these methods, the product can be converted into a suitable salt by a known method, or if the protein ((poly) peptide) is obtained in a salt form, it can be converted into a free form by a known method.

For synthesis of amide derivative of FM-3 and Neuromedin U, usually commercially available resin for protein synthesis can be used. Such resin includes, for example, chloromethyl resin, hydroxymethyl resin, benzhydryl amine resin, aminomethyl resin, 4-benzyloxybenzyl alcohol resin, 4-methylbenzhydryl amine resin, PAM resin, 4-hydroxymethylmethylphenylacetamidemethyl resin, polyacrylamide resin, 4-(2',4'-dimethoxyphenyl-hydroxymethyl) phenoxy resin, 4-(2',4'-dimethoxyphenyl-Fmoc aminoethyl)phenoxy resin, and so forth. On the resin described above, each amino acid with the α-amino group and side-chain functional group properly protected is condensed sequentially in accordance with the sequence of the desired peptide by the per se known condensation methods. At the end of the reaction, the protein ((poly) peptide) is cleaved off from the resin while various protecting groups are removed, and the product is subjected to a reaction of forming intramolecular disulfide bonds in a highly dilute solution to give the desired protein ((poly) peptide).

A wide variety of activating reagents usable for protein synthesis can be used for condensation of the protected amino acids described above, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids along with racemization inhibitors (e.g., HOBt, HOOBt) can be added to the resin directly or after the protected amino acids were previously activated as symmetric acid anhydrides or HOBt esters or HOOBt esters. The solvent used for activation of each protected amino acid or for condensation thereof with the resin can be selected as necessary from those solvents known to be usable in protein ((poly) peptide) condensation reaction. Examples of such solvent include acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; tertiary amines such as pyridine; ethers such as dioxane and tetrahydrofuran; nitriles such as acetonitrile and propionitrile; esters such as methyl acetate and ethyl acetate, or a suitable mixture thereof. The reaction temperature is usually selected as necessary within the range known to be usable in the reaction of forming peptide bonds, and usually the reaction temperature is selected within the range of about -20 °C to 50 °C. The activated amino acid derivatives are used usually in excess (1.5- to 4-fold). When their condensation is insufficient as a result of a test using ninhydrin reaction, their sufficient condensation is achieved by repeatedly carrying out the condensation reaction without conducting elimination of the protecting groups. When their sufficient condensation is not achieved even by repeatedly carrying out the reaction, the unreacted amino acids are acetylated with acetic anhydride or acetyl imidazole so that the subsequent reaction cannot be influenced.

The protecting groups for amino groups in amino acids as the starting materials include, for example, Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosfinothioyl, Fmoc etc.

The carboxyl group can be protected by, for example, described as R in the above, $C_{1-6}$ alkyl, $C_{3-8}$ cycloalkyl, $C_{7-14}$ aralkyl, or 2-adamantyl, 4-nitrobenzyl, 4-methoxybenzyl, 4-chlorobenzyl, phenacyl, benzyloxycarbonylhydrazidation, t-butoxycarbonylhydrazidation, tritylhydrazidation etc.

The hydroxyl group in serine and threonine can be protected by, for example, esterification or etherification. A suitable group used in this esterification includes, for example, lower alkanoyl groups such as acetyl group; alloyl groups such as benzoyl group; and carbonic acid-derived groups such as benzyloxycarbonyl group and ethoxycarbonyl group. A suitable group for etherification includes, for example, a benzyl group, tetrahydropyranyl group, t-butyl group etc.

The protecting group used for the phenolic hydroxyl group in tyrosine includes, for example, Bzl, $Cl_2$-Bzl, 2-nitrobenzyl, Br-Z, t-butyl etc.

The protecting group used for imidazole in histidine includes, for example, Tos, 4-methoxy-2,3,6-trimethylbenzenesulfonyl, DNP, benzyloxymethyl, Bum, Boc, Trt, Fmoc etc.

Activated carboxyl groups in the starting materials include, for example, the corresponding acid anhydrides, azides and active esters (i.e. esters with alcohols such as pentachlorophenol, 2,4,5-trichlorophenol, 2,4-dinitrophenol, cyanomethyl alcohol, p-nitrophenol, HONB, N-hydroxysuccinimide, N-hydroxyphthalimide and HOBt). The activated amino groups in the starting materials include, for example, the corresponding phosphoric acid amides.

Examples of methods for removing (leaving) of the protecting groups include catalytic reduction in a hydrogen stream in the presence of a catalyst such as Pd-black or Pd-carbon; acid treatment using anhydrous hydrogen fluoride, methane sulfonic acid, trifluoromethane sulfonic acid, trifluoroacetic acid or a mixed solution thereof; base treatment using diisopropylethylamine, triethylamine, piperidine or piperazine; and reduction using sodium in liquid ammonia. The leaving reaction by the acid treatment described above is carried out generally at a temperature of about -20 °C to 40 °C, and it is effective in the acid treatment to add a cation scavenger such as anisole, phenol, thioanisole, m-cresol, p-cresol, dimethylsulfide, 1,4-butanedithiol and 1,2-ethanedithiol. A 2,4-dinitrophenyl group used as a protecting group for imidazole in histidine can also be removed by treatment with thiophenol, while a formyl group used as a protecting group for indole in tryptophan can be removed not only by deprotection by acid treatment in the presence of 1,2-ethanedithiol or 1,4-butanedithiol above, but also by alkali treatment using dilute sodium hydroxide solution or dilute ammonia.

Protection and protecting groups for functional groups which should not participate in the reaction of the starting materials, elimination of the protecting groups, and activation of functional groups participating in the reaction can be selected as necessary from known groups or known means.

Another method of obtaining an amide derivative of FM-3 and Neuromedin U includes, for example, amidating the α-carboxyl group of a C-terminal amino acid, then extending a peptide chain at the side of the amino group until it attains desired chain length, and thereafter producing a peptide of said peptide chain from which only the protecting group for the N-terminal α-amino group was removed and a peptide of said peptide chain from which only the protecting group for the C-terminal carboxyl group was removed, followed by condensation both the proteins in the mixed solvent as described above. The details of the condensation reaction are the same as described above. The protected protein obtained by condensation is purified, and every protecting group is removed by the method descried above, whereby the desired crude protein ((poly) peptide) can be obtained. This crude protein ((poly) peptide) is purified by a wide variety of known purification techniques, and by lyophilizing its major fraction, the desired amide derivative of the protein ((poly) peptide) can be obtained.

To obtain an ester derivative of FM-3 and Neuromedin U, for example the α-carboxyl group of a C-terminal amino acid is condensed with desired alcohol to form an amino acid ester from which the desired ester derivative of the protein ((poly) peptide) can be obtained in the same manner as for the amide derivative of the protein((poly) peptide).

The Neuromedin U derivatives used in the present invention may be any peptide having a binding property with FM-3 such as (1) a partial peptide of Neuromedin U; (2) a peptide of which the constitutive amino acid of Neuromedin U was deleted, a peptide to which other amino acids is added to the constitutive amino acid, a peptide in which the constitutive amino acid is substituted by other amino acids; or (3) a labeled Neuromedin U, a labeled partial peptide described in (1) above or a labeled peptide described in (2) above.

Concretely, the partial peptide of Neuromedin U include the peptide having the amino acid sequence represented by SEQ ID NO:16, its amide derivatives, its ester derivatives or a salt thereof. Among those, the amide derivatives of the peptide having the amino acid sequence represented by SEQ ID NO:16 or its salt are preferred.

The partial peptide of said Neuromedin U can be produced by cleaving the Neuromedin U of the present invention with a suitable peptidase or according to the above-mentioned protein ((poly) peptide) synthesis method. The amide derivatives and the ester derivatives of the partial peptide of Neuromedin U can be produced according to the above-mentioned amide derivative production method or the above-mentioned ester derivative production method. Moreover, the salt of the partial peptide of Neuromedin U includes the same salts as the above-mentioned salt of FM-3 and Neuromedin U.

The peptide wherein any of the constitutive amino acids of Neuromedin U is deleted, the peptide wherein other amino acids are added to the constitutive amino acids, the peptide wherein any of the constitutive amino acids is substituted by other amino acids includes, for example, peptides having the amino acid sequence represented by SEQ ID NO:11, of which 1 to 3, preferably 1 or 2 amino acids are deleted, the amino acid sequence represented by SEQ ID NO:11, to which 1 to 3, preferably 1 or 2 amino acids are added (or inserted), or the amino acid sequence represented by SEQ ID NO:11, in which 1 to 3, preferably 1 or 2 amino acids are substituted with other amino acids.

Furthermore, the peptide wherein any of the constitutive amino acids of Neuromedin U is deleted, the peptide wherein other amino acids are added to the constitutive amino acids, the peptide wherein any of the constitutive amino acids is substituted by other amino acids includes, for example, the peptides having the amino acid sequence represented by SEQ ID NO:5, SEQ ID NO:6, SEQ ID NO:7, SEQ ID NO:8, SEQ ID NO:9, SEQ ID NO:10, SEQ ID NO:12, SEQ ID NO:13, SEQ ID NO:14 or SEQ ID NO:15, of which 1 to 3, preferably 1 or 2 amino acids are deleted, the amino acid sequence represented by SEQ ID NO:5, SEQ ID NO:6, SEQ ID NO:7, SEQ ID NO:8, SEQ ID NO:9, SEQ ID NO: 10, SEQ ID NO:12, SEQ ID NO:13, SEQ ID NO:14 or SEQ ID NO:15, to which 1 to 3, preferably 1 or 2 amino acids are added (or inserted), or the amino acid sequence represented by SEQ ID NO:5, SEQ ID NO:6, SEQ ID NO:7, SEQ ID NO:8, SEQ ID NO:9, SEQ ID NO:10, SEQ ID NO:12, SEQ ID NO:13, SEQ ID NO:14 or SEQ ID NO:15, in which 1 to 3, preferably 1 or 2 amino acids are substituted with other amino acids.

The substantially same substituent as the amino acid in said amino acid sequence can be selected from, for example, any other amino acids of the group that the amino acid belongs to. Non-polar (hydrophobic) amino acid includes alanine, leucine, isoleucine, valine, proline, phenylalanine, tryptophan, methionine, etc. Polar (neutral) amino acid includes glycine, serine, threonin, cycteine, thyrosine, asparagine, glutamine, etc. Positively charged (basic) amino acid includes arginine, lysine, histidine, etc. Negatively charged (acidic) amino acid includes aspartic acid, glutamic acid, etc.

The labeled Neuromedin U, the labeled partial peptide described (1) above and the labeled peptide described (2) above include peptides which may be labeled in a publicly known manner. Specific examples include those labeled with an isotope, those labeled with fluorescence (e.g. fluorescein), those biotinated, and those labeled with enzyme.

Concretely, the Neuromedin U labeled with the radioisotope such as [$^{3}$H], [$^{125}$I], [$^{14}$C], [$^{35}$S] in a publicly known manner is used.

The salt of Neuromedin U derivatives is the same salt as those salts of FM-3 and Neuromedin U mentioned above.

The DNA encoding FM-3 used in the present invention may be any of DNA encoding the protein having the same or the substantially same amino acid sequence as that represented by SEQ ID NO:1, and the DNA encoding Neuromedin U used in the present invention may be any of DNA encoding peptide having the same or the substantially same amino acid sequence as that represented by SEQ ID NO:11. The DNA encoding FM-3 or Neuromedin U may be derived from any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA fraction prepared from the cells and tissues described above.

The DNA encoding FM-3 having the amino acid sequence represented by SEQ ID NO:1 includes, for example, the DNA having the base sequence represented by SEQ ID NO:2. The DNA encoding FM-3 having the amino acid sequence represented by SEQ ID NO:3 includes, for example, the DNA having the base sequence represented by SEQ ID NO:4. The DNA encoding FM-3 having the amino acid sequence represented by SEQ ID NO:19 includes the DNA having the base sequence represented by SEQ ID NO:20.

Concretely, the following DNA is used: (1) the DNA hybridizing under high stringent conditions with the DNA encoding the protein or (poly)peptide having the same or the substantially same amino acid sequence as that represented by SEQ ID NO:1 or SEQ ID NO:11, (2) the DNA encoding the protein or (poly)peptide having the same or the substantially same amino acid sequence as that represented by SEQ ID NO:1 or SEQ ID NO:11, which does not hybridize with the DNA sequence encoding the protein or (poly) peptide having the same or the substantially same amino acid sequence represented by SEQ ID NO:1 or SEQ ID NO:11 or the sequence determined in (1) due to the degeneracy of genetic code. Hybridization can be carried out according to a per se known method. The high stringent conditions used herein refer to the conditions, for example, 50% formaldehyde, 4xSSPE (1x SSPE=150mM NaCl, 10mM $NaH_2PO_4/H_2O$, 1mM EDTA, pH7.4), 5x Denhardt's solution and 0.1% of SDS at a temperature of 42°C.

The DNA encoding FM-3 or Neuromedin U used in the present invention can be produced according to a genetic engineering method described below.

For cloning the complete DNA encoding FM-3 or Neuromedin U of the present invention, the desired DNA may be either amplified by per se known PCR method using synthetic DNA primers containing the base sequence encoding the partial peptide of polypeptide of the present invention from the above-mentioned DNA library, or the DNA inserted into an appropriate vector can be selected by hybridization with a labeled DNA fragment or synthetic DNA that encodes a part or entire region of the FM-3 or Neuromedin U. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the ODA-LA PCR method, the Gupped duplex method or the Kunkel method or its modification using a publicly known kit available as Mutan™-super Express Km or Mutan™-K (both manufactured by Takara Shuzo Co., Ltd.).

The cloned DNA encoding FM-3 or Neuromedin U used in the present invention can be used depending upon purpose, as it is or if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for FM-3 or Neuromedin U used in the present invention can be manufactured, for example, by (a) excising the desired DNA fragment from the DNA encoding FM-3 or Neuromedin U of the present invention, and then (b) ligating the DNA fragment with an appropriate expression vector downstream of a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. The promoter used in the present invention may be any promoter if it matches well with a host to be used for gene expression.

In the case of using animal cells as the host, examples of the promoter include SV40 promoter, a retrovirus promoter, a metallothionein promoter, a heat shock promoter, a cytomegalovirus promoter, SRα promoter, etc. Where the host is bacteria of the genus Escherichia, preferred examples of the promoter include trp promoter, T7 promoter, lac promoter, recA promoter, $\lambda P_L$ promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH1 promoter, GAL promoter. When insect cells are used as the host, preferred examples of the promoter include polyhedrin prompter and P10 promoter.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$),

neomycin resistant gene (hereinafter sometimes abbreviated as $Neo^r$, G418 resistance), etc. In particular, when dhfr gene is used as the selection marker in CHO ($dhfr^-$) cell selection can also be made on thymidine free media.

If necessary and desired, a signal sequence that matches with a host is added to the N-terminus of the polypeptide and the partial peptide. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, invertase signal sequence, etc. in the case of using yeast as the host; and insulin signal sequence, α-interferon signal sequence, antibody molecule signal sequence, etc. in the case of using animal cells as the host, respectively.

Using the vector containing the DNA encoding FM-3 or Neuromedin U of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, $AH22R^-$, NA87-11A, DKD-5D, 20B-12, etc.

Examples of insect include a larva of Bombyx mori (Maeda, et al., Nature, 315, 592 (1985)).

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vitro, 13, 213-217 (1977).

Examples of animal cells include monkey cells COS-7, Vero cells, Chinese hamster cells CHO (hereinafter referred to as CHO cells), dhfr gene deficient Chinese hamster cells CHO (hereinafter simply referred to as CHO(dhfr) cell), mouse L cells, mouse 3T3, mouse myeloma cells, human HEK293 cells, human FL cells, 293 cells, C127 cells, BALB3T3 cells, Sp-2/O cells, etc.

Bacteria belonging to the genus Escherichia can be transformed, for example, by the method described in Proc. Natl. Acad. Sci. U.S.A., 69, 2110 (1972) or Gene, 17, 107 (1982). Bacteria belonging to the genus Bacillus can be transformed, for example, by the method described in Molecular & General Genetics, 168, 111 (1979).

Yeast can be transformed, for example, by the method described in Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Virology, 52, 456 (1973).

The method of introduction of the expression vector into the cell includes, for example, lipofection (Felgner, P.L. et al. Proc. Natl. Acad. Sci. U.S.A., 84, 7413 (1987)), Calcium phosphate method (Graham, F.L. and van der Eb, A.J. Virology, 52, 456-467 (1973)), electroporation (Nuemann, E. et al. Embo J., 1, 841-845 (1982)) are listed.

Thus, the transformant transformed with the expression vector containing the DNA encoding FM-3 or Neuromedin U can be obtained.

Furthermore, the method of expressing FM-3 or Neuromedin U used in the present invention in a stable manner using animal cells includes a method of screening the animal cells wherein the expression vector introduced to the cells is incorporated into the chromosome by a clonal selection. To be more specific, using the above selection marker as an index, a transformant can be selected. For those animal cells obtained by use of the selection marker, it is possible to obtain a stable animal cell strain having a highly expressed FM-3 or Neuromedin U used in the present invention by repeating the clonal selection. Moreover, in case of using dhfr gene as a selection marker, the cells are cultured with the gradually increased concentration of MTX, and the resistant cell strain is selected. In this way, it is possible to obtain the highly expression animal cell strain by amplifying the DNA encoding FM-3 or Neuromedin U in the cell with dhfr gene.

FM-3 or Neuromedin U used in the present invention can be manufactured by cultivating the above-mentioned transformant under condition capable of expressing the DNA encoding FM-3 or Neuromedin U used in the present invention; and producing and accumulating FM-3 or Neuromedin U used in the present invention.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc.

Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium to activate the promoter efficiently thereby.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as 10% inactivated bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), which contain about 5% to about 20% fetal bovine serum. Preferably, pH of the medium is adjusted to about 6 to about 8. The transformant is usually cultivated at about 30°C to about 40°C for about 15 hours to about 60 hours and, if necessary and desired, the culture can be aerated or agitated.

In the case of using CHO(dhfr$^-$)cells and dhfr gene as a selection marker, DMEM medium containing thymidine-free dialyzed fetal bovine serum is preferred to use.

FM-3 or Neuromedin U used in the present invention can be separated and purified from the culture described above by the following procedures.

When FM-3 or Neuromedin U used in the present invention is extracted from the cultured transformants or cells, after cultivation, the transformants or cells are collected by a publicly known method, suspended in a appropriate buffer, and then disrupted by publicly known methods such as ultrasonication, a treatment with lysozyme and/or freeze-thaw cycling, followed by centrifugation, filtration, etc. Thus, the crude extract of FM-3 or Neuromedin U used in the present invention can be obtained. The buffer used for the extraction may contain a protein denaturizing agent such as urea or guanidine hydrochloride, or a surfactant such as Triton X-100™, etc.

When FM-3 or Neuromedin U used in the present invention is secreted to the culture medium, after the cultivation, the transformants or cells can be separated to collect the supernatant by a publicly known method.

FM-3 or Neuromedin U contained in the supernatant or the extract thus obtained can be purified by an appropriate combination of the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing, chromatofocusing; and the like.

When FM-3 or Neuromedin U used in the present invention is obtained in a free form, it can be converted into the salt by publicly known methods or modifications thereof. On the other hand, when FM-3 or Neuromedin U is obtained in a salt form, it can be converted into the free form or another salt form by publicly known methods or modifications thereof.

FM-3 or Neuromedin U used in the present invention produced by the recombinant can be treated, before or after the purification, with an appropriate protein modifying enzyme so that FM-3 or Neuromedin U can be appropriately modified or be deprived of a partial (poly)peptide. Examples of the protein-modifying enzyme include trypsin, chymotrypsin, arginyl endopeptidase, protein kinase, glycosidase or the like. It is possible to use publicly known Edman method using Edman reagent (phenyl iso-thiocyanate) to delete the N-terminal amino acid.

The presence of the thus produced FM-3 or Neuromedin U used in the present invention can be determined by an enzyme immunoassay using a specific antibody, or the like.

The details of the screening method for the compound or its salt that alters the binding property between Neuromedin U and FM-3, characterized by using Neuromedin U and FM-3, and the screening kit for the compound or its salt that alters the binding property between Neuromedin U and FM-3, characterized by containing Neuromedin U and FM-3, are described below.

Using the binding assay system (ligand/receptor assay system) of Neuromedin U with FM-3 or the constructed recombinant FM-3 expression system, the compound that alters the binding property between Neuromedin U and FM-3 (e.g., peptides, proteins, non-peptide compounds, synthetic compounds, fermentation products, etc.) or its salt can be screened.

These compounds include the compound(FM-3 agonist)containing cell stimulating activities through FM-3 (e.g., the activities to enhance arachidonic acid release, acetylcholine release, intracellular $Ca^{2+}$ release, intracellular cAMP production, intracellular cGMP production, inositol phosphate production, cell membrane potential change, phosphorylation of intracellular proteins, c-fos activation and pH change), and the compound(FM-3 antagonist) containing no such cell stimulating activities.

The wording "alter the binding property between Neuromedin U and FM-3" means either of properties of inhibiting or enhancing the binding between Neuromedin U and FM-3 (prolonging the binding time).

That is, the present invention provides:

A method for screening the compound or a salt thereof that alters the binding property of Neuromedin U with FM-3, characterized by comparing the case (i) where Neuromedin U is brought in contact with FM-3 and (ii) where Neuromedin U and a test compound are brought in contact with the above-mentioned FM-3.

In the screening method of the present invention, for example, a binding amount of the ligand with the FM-3 and a level of cell stimulating activity are measured and compared (i) where Neuromedin U is brought in contact with above-mentioned FM-3 and (ii) where Neuromedin U and a test compound are brought in contact with the above-mentioned FM-3.

Concretely, the screening method of the present invention is:

(1) A method for screening a compound or a salt thereof that alters the binding property between Neuromedin U and FM-3, which comprises measuring and comparing the binding amounts of a labeled Neuromedin U (as one of the above-mentioned Neuromedin derivative)(hereinafter simply referred to as "labeled Neuromedin U") with FM-3, where a labeled Neuromedin U is brought in contact with the above-mentioned FM-3 and where a labeled Neuromedin U and a test compound are brought in contact with the FM-3;

(2) A method for screening a compound or a salt thereof that alters the binding property between Neuromedin U and FM-3, which comprises measuring and comparing the binding amounts of a labeled Neuromedin U with cells containing FM-3 or a membrane fraction of the cells, where a labeled Neuromedin U is brought in contact with cells containing FM-3 or a membrane fraction of the cells and where a labeled Neuromedin U and a test compound are brought in contact with cells containing FM-3 or a membrane fraction of the cells;

(3) A method for screening a compound or a salt thereof that alters the binding property between Neuromedin U and FM-3, which comprises measuring and comparing the binding amounts of a labeled Neuromedin U with FM-3, where a labeled Neuromedin U is brought in contact with FM-3 expressed on the cell membrane by culturing a transformant containing DNA encoding FM-3 and where a labeled Neuromedin U and a test compound are brought in contact with FM-3 expressed on the cell membrane by culturing a transformant containing DNA encoding FM-3;

(4) A method for screening a compound or a salt thereof that alters the binding property between Neuromedin U and FM-3, which comprises measuring and comparing cell stimulating activities through FM-3 (e.g., the activities to enhance arachidonic acid release, acetylcholine release, intracellular $Ca^{2+}$ release, intracellular cAMP production, intracellular cGMP production, inositol phosphate production, change in cell membrane potential, phosphorylation of intracellular proteins, c-fos activation and pH change), where a compound which activates FM-3 (e.g. Neuromedin U) is brought in contact with cells containing FM-3 and where a compound which activates FM-3 and a test compound are brought in contact with cells containing FM-3; and

(5) A method for screening a compound that or a salt thereof alters the binding property between Neuromedin U and FM-3, which comprises measuring and comparing cell stimulating activities through FM-3 (e.g., the activities to enhance arachidonic acid release, acetylcholine release, intracellular $Ca^{2+}$ release, intracellular cAMP production, intracellular cGMP production, inositol phosphate production, cell membrane potential change, phosphorylation of intracellular proteins, c-fos activation and pH change), where a compound which activates FM-3 (e.g. Neuromedin U) is brought in contact with FM-3 expressed on the cell membrane by culturing a transformant containing DNA encoding FM-3 and where a compound which activates FM-3 and a test compound are brought in contact with FM-3 expressed on the cell membrane by culturing a transformant containing DNA encoding FM-3.

Thus, the concrete description of the screening methods of the present invention is as follows:

For the FM-3 used in the screening method of the present invention, any substance may be used so long as it contains the above-mentioned FM-3. The cell membrane fraction from organs of human, warm-blooded animal, amphibian or fish is preferred. However, it is very difficult to obtain, in particular human organs. It is thus preferable to use FM-3 produced by large-scale expression using recombinants.

To produce FM-3, the above-mentioned method is applied.

In the screening methods, the cells containing FM-3 or the cell membrane fraction can be prepared according to the preparation method described below.

Where cells containing FM-3 are used, the cells may be fixed using glutaraldehyde, formalin, etc. The fixation can be made by a publicly known method.

The cells containing FM-3 include host cells that have expressed FM-3. Such host cells include Escherichia coli, Bacillus subtilis, yeast, insect cells, animal cells, and the like.

The cell membrane fraction refers to a fraction abundant in cell membrane obtained by cell disruption and subsequent fractionation by a publicly known method. The cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, the disrupted cell solution is centrifuged at a low speed (500 rpm to 3,000 rpm) for a short period(normally about 1 to 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in expressed FM-3, cell-derived phospholipids and membrane proteins.

The amount of FM-3 in the cells containing FM-3 and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (1) through (3) described above for screening a compound that alters the binding property between Neuromedin U and FM-3, an appropriate FM-3 fraction, a labeled ligand or a compound having a ligand activity (e.g. Neuromedin U and a salt thereof) are used. FM-3 fraction is preferably a fraction of a naturally occurring receptor protein or a recombinant receptor protein having an activity equivalent to that of the natural protein. Herein, the equivalent activity is intended to mean a ligand binding activity. For the labeled ligand, a labeled ligand and a compound having a ligand activity (e.g. Neuromedin U and a derivative thereof) are used. For example, ligands labeled with [$^3$H], [$^{125}$I], [$^{14}$C], [$^{35}$S], etc. (the labeled Neuromedin U as a Neuromedin U derivative) are used.

More specifically, to conduct the screening for the compound that alters the binding property between Neuromedin U and FM-3, first, a receptor preparation is prepared by suspending cells containing FM-3 or the membrane fraction thereof in a buffer appropriate for the screening method. Any buffer can be used so long as it does not inhibit the ligand-receptor binding, such buffers including a phosphate buffer or a Tris-HCl buffer having pH of 4 to 10 (preferably pH of 6 to 8). For the purpose of minimizing non-specific binding, a surfactant such as CHAPS, Tween-80™ (manufactured by Kao-Atlas Inc.), digitonin or deoxycholate, may optionally be added to the buffer. Further for the purpose of suppressing the degradation of FM-3 and Neuromedin U by proteases, a protease inhibitor such as PMSF, leupeptin, E-64 (manufactured by Peptide Institute, Inc.) and pepstatin may also be added. A given amount (5,000 to 500,000 cpm) of labeled Neuromedin U (labeled Neuromedin as Neuromedin U derivatives) is added to 0.01 ml to 10 ml of the receptor solution. Also, $10^{-4}$ to $10^{-1}$ μM of the test compound are added to the mixture of Neuromedin U and the receptor solution. To determine the amount of non-specific binding (NSB), a reaction tube containing an unlabeled test compound in large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. Regarding the count obtained by subtracting the amount of non-specific binding (NSB) from the count obtained in the absence of any competitive substance ($B_0$) as 100%, the test compound, which makes the amount of specific binding (B-NSB), for example 80% or less, can be selected as the compound that is capable to alter the binding property between FM-3 and Neuromedin U.

As the method for measuring the binding between FM-3 and Neuromedin U, BIAcore (Amasham pharmacia Biotech) may be used. In this method, Neuromedin U is fixed to a sensor chip according to amino coupling method described in the protocol that is attached to the device. A buffer(such as phosphate buffer and Tris buffer) solution containing FM-3 purified from the cells having FM-3 or a transformant having the DNA encoding FM-3, or a membrane fraction having FM-3, or a buffer solution containing a purified FM-3 or a membrane fraction having FM-3 and a test compound is passed through the top of the sensor chip at 2-20 μ l/min By observing the co-existing test compound to alter the surface plasmo resonance change which occurs by binding FM-3 and Neuromedin U on the sensor chip, the compound that alters the binding property between FM-3 and Neuromedin U can be screened. This method can also be carried out by fixing FM-3 to the sensor chip and passing the buffer solution(such as phosphate buffer or Tris buffer) containing Neuromedin U and a test compound through the top of the sensor chip. The test compounds include those as described above, etc.

To perform the above screening methods (4) and (5), the compound that alters the binding property between Neuromedin U and FM-3, the FM-3-mediated cell-stimulating activity (e.g., activity that promotes or inhibits arachidonic acid release, acetylcholine release, intracellular $Ca^{2+}$ release, intracellular cAMP production, intracellular cGMP production, inositol phosphate production, cell membrane potential change, phosphorylation of intracellular proteins, c-fos activation, pH change, etc.) can be measured using per se known methods or commercially available measuring kits. Concretely, the cells containing FM-3 are first cultured on a multi-well plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

Screening by assaying the cell-stimulating activity requires cells that have expressed an appropriate FM-3. For the cells that have expressed FM-3, the cell line expressing the recombinant FM-3 described above and the like are desirable. The transformed cells capable of expressing FM-3 can be either a stable expression strain or a transient expression strain. For animal cells, the same kinds of cells described above are used.

For the test compound, for example, peptides, proteins, non-peptide compounds, synthetic compounds, fermentation products, cell extracts, plant extracts, and animal tissue extracts are used.

To describe the above-mentioned ligand/receptor assay more specifically, the following assay systems and the like are used:

(1) When a receptor-expressing cell is stimulated by a receptor agonist, an intracellular G-protein becomes active and, as a result, GTP bonds with it. The same phenomena can be observed with a cell membrane of receptor expression cell. Generally, GTP is converted to GDP by hydrolysis. When GTPγS is added to the reaction solution, GTPγS bonds with G-protein as GTP does, and it does not suffer from hydrolysis with keeping the binding to the cell membrane containing the G-protein. Using the labeled GTPγS, it is possible to measure the receptor expression cell stimulating activity of the receptor agonist by measuring the radioactivity remaining in the cell membrane. Applying this reaction, a stimulating activity of Neuromedin U with respect to FM-3-expressing cells can be measured. This method does not use cells containing FM-3 as described above (4)-(5). This method is an assay using the cell membrane containing FM-3 as described in (1)-(3), and is an assay to measure a cell stimulating activity as described in (4) - (5). In this assay, a substance which shows an activity to promote the binding of GTPγ S to FM-3-containing cell membrane fraction is an agonist. By adding Neuromedin U or Neuromedin U and a test compound and observing the change in GTPγS binding acceleration activity to a FM-3-containing cell membrane fraction as compared with a single administration of Neuromedin U, the compound that alters the binding property between Neuromedin U and FM-3 can be screened. The compound which indicates the activity that inhibits the GTPγS binding acceleration activity to a FM-3-containing cell membrane fraction by Neuromedin U can be selected as a candidate substance which is capable of altering the binding property between FM-3 and Neuromedin U. On the other hand; an agonist can be screened by adding a test compound alone and observing the GTPγS binding acceleration activity to a FM-3-containing cell membrane fraction as well.

Concretely, an example of the screening methods is described as follows. A cell membrane fraction containing FM-3 prepared by the method described above is diluted with a membrane dilution buffer solution (e.g. 50mM Tris, 5mM $MgCl_2$, 150mM NaCl, 1μ GDP, 0.1% BSA pH7.4). The dilution scale may vary according to the amount of receptor expression. 0.2 ml of the solution is transferred to Falconb 2053. Neuromedin U or Neuromedin U and a test compound are added thereto, and then [$^{35}$S]GTP γ S is added to make the final concentration of 200pM. After the mixture is kept at 25°C for an hour, an ice-cold buffer solution for washing (50mM Tris, 5mM $MgCl_2$, 150mM NaCl, 0.1% BSA, 0.05% CHAPS pH7.4 1.5ml) is added. Then, the solution is filtered with a glass fiber filtering paper GF/F. After drying the filtering paper at 65°C for 30 min., the radioactivity of [$^{35}$S]GTP γ S bound with the membrane fraction left on the filtering paper is measured on a liquid scintillation counter. The radioactivity in the experiment with a single administration of Neuromedin U is set as 100%, the radioactivity in the experiment without adding Neuromedin U is set as 0%, and an influence of a test compound to the GTPγS binding acceleration activity by Neuromedin U is calculated. A test compound which makes GTPγS binding acceleration activity, for example 80 % or less, can be selected as a candidate substance which is capable of altering the binding property between FM-3 and Neuromedin U.

(2) When the amount of intracellular cAMP is reduced by the Neuromedin U stimulation in a FM-3-expressing cell, using this reaction, the cell stimulating activities of Neuromedin to a FM-3-expressing cell can be measured. Using the anti-cAMP antibody obtained by immunized mice, rats, rabbits, goats and cows and $^{125}$I-labeled cAMP (both are commercially available), the amount of cAMP production in various animal cells expressing FM-3 can be measured by RIA or other EIA system such as the combination of anti-cAMP antibody and the labeled cAMP. It is also possible to conduct a quantification by the SPA method using beads containing the scintillant to which an anti-cAMP is fixed using Protein A or an antibody to IgG of an animal used for production of the anti-cAMP antibody, and $^{125}$I-labeled cAMP (using the kit manufactured by Amasham pharmacia Biotech).

In this assay system, it is possible to conduct a screening of the compound that alters the binding property of Neuromedin U and FM-3 by increasing the amount of intracellular cAMP by ligand such as Calcitonin and Forskolin which increase the amount of intracellular cAMP; adding Neuromedin U or Neuromedin U and the test compound; and observing the change in the amount of intracellular cAMP as compared to the case with a single administration of Neuromedin U. Then, the compound that indicates an activity that inhibits the cAMP production inhibition activity of the FM-3-expressing cells by Neuromedin U can be selected as a candidate substance that is capable of altering the binding property between FM-3 and Neuromedin U. On the other hand, the compound that indicates an agonist activity can be screened by adding the test compound alone and measuring the cAMP production inhibition activity.

More specifically, the screening methods are described as follows. CHO cells expressing FM-3 are plated at $5x10^4$ cell/well on a 24-well plate, and cultivated for about 48 hours. The cells are washed with Hanks' buffer containing 0.2mM 3-isobutyl-methylxanthine, 0.05% BSA and 20mM HEPES (pH7.4) (hereinafter referred to as reaction buffer). Then, 0.5ml of the reaction buffer is added to the cells, and the cells are kept in an incubator for 30 minutes. Then, the reaction buffer is removed and 0.25ml of fresh reaction buffer is added to the cells. Then, the reaction buffer (0.25ml) containing 2μM Forskolin in addition to 1nM of Neuromedin U or Neuromedin U and a test compound is added to the cells. The reaction is made at 37°C for 24 minutes. 100μl of 20% Perchloric acid is added to stop the reaction. Then, by placing it on ice, the intracellular cAMP is extracted. The amount of cAMP in the extraction is measured by using cAMP EIA kit (Amasham pharmacia biotech). The amount of cAMP produced by the Forskolin stimulation is set as 100%, the amount of cAMP inhibited by the addition of 1nM of Neuromedin is set as 0% and an influence of the test compound on the cAMP production inhibition activity by Neuromedin U is calculated. A test compound which makes the cAMP production activity, for example, 80% or less by inhibiting the Neuromedin U activity can be selected as a candidate substance that is capable of altering the binding property between FM-3 and Neuromedin U.

To measure the cAMP production acceleration activity, the cAMP produced by adding a test compound to the FM-3 expression CHO cells without added Forskolin is measured according to the above-mentioned method. In this case, a test compound which makes the cAMP production activity, for example, 10% or more, can be selected as a candidate substance that is capable of altering the binding property between FM-3 and Neuromedin U.

(3) The DNA containing CRE (cAMP response element) is inserted into the multi-cloning site upstream of luciferase gene of Picagene basic vector or Picagene enhancer vector (Toyo Ink). It is named as CRE-reporter gene vector. In the cell transfected with the CRE-reporter gene vector, a stimulation which causes the increase in cAMP, induces an expression of luciferase gene through CRE and a production of luciferase protein. By measuring the luciferase activity, it is possible to detect the change in the amount of cAMP in the cells into which the CRE-reporter gene vector is introduced. Thus, the compound that alters the binding property of Neuromedin U and FM-3 can be screened using the FM-3-expressing cells to which the CRE-reporter gene vector is transfected. The details of the screening method are as follows.

CRE-reporter gene introduced FM-3-expressing cells is placed in a 24-well plate at a concentration of $5x10^3$ cell/well, and cultivated for about 48 hours. The cells are washed with Hanks' buffer (pH7.4) containing 0.2mM 3-isobutyl-methyl xanthine, 0.05% BSA and 20mM HEPES (hereinafter, Hanks' buffer(pH7.4) containing 0.2mM 3-isobutyl-methyl xanthine, 0.05% BSA and 20mM HEPES, is referred to as reaction buffer). 0.5ml of the reaction buffer is added to the cells. Then, the cells are kept warm in a cultivator for 30 minutes. Then, the reaction buffer is removed from the system. 0.25ml of fresh reaction buffer is added to the cells. Then, the reaction buffer 0.25ml containing 2μ M Forskolin in addition to 1nM of Neuromedin U or Neuromedin U and a test compound is added to the cells. The reaction is made at 37°C for 24 minutes. The cells are dissolved in a decomposition solution for Picagene (Toyo Ink). To the decomposition solution, a luminescent substance (Toyo Ink) is added. The luminescence by luciferases is measured with a luminometer, a liquid scintillation counter, a top counter or the like. An influence of the compound that alters the binding property of Neuromedin U and FM-3 can be measured by comparing the luminescence by luciferases with the case where Neuromedin U is singly administrated. In this process, by administrating Neuromedin U, the increase of luminescence by the Folskolin stimulation is inhibited. The compound that recovers the influence of Neuromedin U, may be selected as a candidate substance that alters the binding property between Neuromedin U and FM-3. On the other hand, an agonist can be screened by adding the test compound singly and observing the inhibition of the increase in luminescence caused by the Folskolin stimulation, as Neuromedin U inhibits the increase.

Alkaline phosphatase, chloramphenicol, acetyltransferase or β-galactosidase can be used as a reporter gene, besides luciferase. The activity of the product of reporter gene can be measured easily using commercially available measuring kit. The activity of alkaline phosphatase can be measured by Lumi-Phos 530(Wako); the activity of Chloramphenicol and acetyltransferase can be measured by FAST CAT chrolamphenicol Acetyltransferase Assay Kit(Wako); and the activity of β-galactosidase can be measured by Aurora Gal-XE (Wako).

(4) When FM-3-expressing cells release the metabolic substance of arachidonic acid to the outside by the Neuromedin stimulation, if arachidonic acid having radioactivity is taken into the cell beforehand, it is possible to measure a cell stimulating activity by measuring the radioactivity released out of the cells. In this process, by adding Neuromedin U or Neuromedin U and a test compound and examining an influence of Neuromedin U on the arachidonic acid metabolite release activity, the compound that alters the binding property between Neuromedin U and FM-3 can be screened. The compound that inhibits the arachidonic acid metabolite release activity of Neuromedin U can be selected as a candidate substance that alters the binding property of Neuromedin U and FM-3. Moreover, the compound that indicates an agonist activity can be screened by adding the test compound singly and checking the arachidonic acid metabolite release activity in FM-3-expressing cells.

The details of the screening method of the compound that has the influence on the binding between Neuromedin U and FM-3 are as follows.

FM-3 expression CHO cells are placed at $5x10^4$ cell/well on a 24-well plate, and cultivated for about 24 hours. After cultivation, 0.25μCi/well of $[^3H]$ arachidonic acid is added. 16 hours after adding $[^3H]$ arachidonic acid, the cells are washed with Hanks' buffer (pH7.4) containing 0.05% BSA and 20mM HEPES. Then, 500μl of the Hanks' buffer (pH7.4) containing 0.05% BSA and 20mM HEPES in the presence of the final concentration of 10nM Neuromedin U or 10nM Neuromedin U and the test compound is added to each well(hereinafter, Hanks' buffer (pH7.4) containing 0.05% BSA and 20mM HEPES is referred to as reaction buffer). After incubating at 37°C for 60 minutes, 400μl of the reaction solution is added to a scintillator. Then, the amount of released $[H^3]$ arachidonic acid metabolite is measured by a scintilation counter. The amount of $[H^3]$ arachidonic acid metabolite in the medium without added Neuromedin U, is set as 0%, the amount of $[H^3]$ arachidonic acid metabolite in the medium with added 10nM Neuromedin, is set as 100%, and an influence of the test compound on the binding of Neuromedin U and FM-3 is calculated. A test compound which makes the arachidonic acid metabolite production activity, for example, 50% or less, can be selected as a candidate substance that is capable of altering the binding property between FM-3 and Neuromedin U.

(5) When Neuromedin U stimulates the increase in intracellular $Ca^{2+}$ concentration in FM-3-expressing cells, using this fact, an influence of test compound on the binding between Neuromedin U and FM-3 can be examined.

FM-3-expressing cells are placed on a sterilized cover glass for a microscope. After 2 days, the medium is replaced with HBSS in which 4mM of Fura-2 AM (Dojin Kagaku) is suspended, and left for 2 and half hours at room temperature. After washing with HBSS, the cover glass is set to a cuvet. The increase in the ratio of intensity of fluorescence at 505nm where the excited wave length is 340nm and 380nm, is measured by a spectrophotofluorometer when Neuromedin U or Neuromedin U and a test compound are added. By measuring the change in the intensity of fluorescence caused by adding the test compound compared with that by the single administration of Neuromedin U, the compound which has the influence on the binding between Neuromedin U and FM-3 can be screened. Furthermore, FLIPR (Manufactured by Molecular device) can be also used as follows. Fluo-3 AM (Manufactured by Dojin Kagaku) is added to the cell suspension to let the cells take up Fluo-3AM. The cells are washed by centrifuging several times, and placed on a 96-well plate. The cells are set to a FLIPR device, and Neuromedin U or Neuromedin U and a test compound are added in the same way as Fura-2AM. By measuring the change in the intensity of fluorescence caused by added the test compound as compared with that by the single administration of Neuromedin U, the compound which has an influence on the binding between Neuromedin U and FM-3 can be screened. Above these, the compound that inhibits the increase in the intensity of fluorescence by Neuromedin U can be selected as a candidate substance that is capable of altering the binding property between FM-3 and Neuromedin U. On the other hand, by observing the increase in the intensity of fluorescence by single administration of the test compound, an agonist can be screened.

To screen the compound that has an influence on the binding between Neuromedin U and FM-3, first, FM-3-expressing cells are allowed to co-express a gene of a protein such as Aequorin which radiates light when the intracellular Ca ion increases. The increase in the intracellular Ca ion causes Aequorin to become Ca binding type and radiates light. Using this fact, Neuromedin U or Neuromedin U and a test compound are added and the change in the intensity of luminescence when the test compound is added as compared with that by a single administration of Neuromedin U is observed for the screening. The method is almost the same as the above-mentioned method except that this method does not require cells to take up a fluorescence substance.

(6) By adding an agonist to receptor-expressing cells, the concentration of inositol triphosphate rises. By observing the reaction in FM-3-expressing cells caused by Neuromedin U, the compound that has an influence on the binding between Neuromedin U and FM-3 can be screened. Cells are placed in a 24-well plate, and incubated for one day, and incubated for one more day in a medium to which myo-[2-$^3$H]inositol (2.5μCi/well) is added. After washing well, Neuromedin U or Neuromedin U and a test compound are added thereto, and then 10% Perchloric acid is added to stop the reaction. The reaction solution is neutralized with 1.5M KOH and 60mM HEPES solution, and passed through a column filled with AG1x8 resin (Bio Rad). After washing with 5mM $Na_2BO_3$ and 60mM $HCOONH_4$, the radioactivity, which is eluted by 1M $HCOONH_4$ and 0.1M HCOOH, is measured by a liquid scintillation counter. The radioactivity in the medium when Neuromedin U is not added, is set as 0%, the radioactivity in the medium when Neuromedin U is added, is set as 100%, and an influence on the binding between Neuromedin U and FM-3 can be calculated. A test compound which makes the inositol triphosphate production activity, for example, 50% or less can be selected as a candidate substance that is capable of altering the binding property between FM-3 and Neuromedin U. On the other hand, by observing the increase in the inositol triphosphate production activity by single administration of the test compound, an agonist can be screened.

(7) The DNA containing TRE (TPA response element) is inserted into the multi-cloning site upstream of luciferase gene of Picagene basic vector or Picagene enhancer vector (Toyo Ink). It is named as TRE-reporter gene vector. In the cell transfected with the TRE-reporter gene vector, a stimulation which causes the increase in intracellular $Ca^{2+}$ induces an expression of luciferase gene through TRE and a production of luciferase protein. By measuring the luciferase activity, it is possible to detect the change in the amount of intracellular calcium in the cells into which the TRE-reporter gene vector is introduced. The details of the screening method of the compound that alters the binding between Neuromedin U and FM-3 using the FM-3-expressing cells to which TRE-reporter gene vector is transfected are as follows.

The TRE-reporter gene introduced FM-3-expressing cells are placed in a 24-well plate at $5x10^3$ cell/well, and cultivated for about 48 hours. The cells are washed with Hanks' buffer(pH7.4) containing 0.05% BSA and 20mM HEPES. 10nM Neuromedin or 10nM Neuromedin U and a test compound are added thereto. Then, the reaction is made at 37C° for 60 minutes. The cells are dissolved in a decomposition solution for Picagene (Toyo Ink). To the decomposition solution, a luminescence substance (Toyo Ink) is added. The luminescence by luciferases can be measured with a luminometor, a liquid scintillation counter, a top counter or the like. An influence of the compound that alters the binding property of Neuromedin U and FM-3 can be measured by comparing the luminescence by luciferases with that when Neuromedin U is singly administrated. In this process, by administrating Neuromedin U, the amount of luminescence increases through the increase in intracellular $Ca^{2+}$. The compound that inhibits the increase may be selected as a candidate substance that alters the binding property between Neuromedin U and FM-3. On the other hand, an agonist can be screened by adding the test compound singly and observing the increase in luminescence like the increase by Neuromedin U.

Alkaline phosphatase, chloramphenicol, acyltransferase or β-galactosidase can be used as a reporter gene, besides luciferase. The activity of the product of reporter gene can be measured easily using commercially available measuring kit. The activity of alkaline phosphatase can be measured by Lumi-Phos 530(Wako); the activity of Chloramphenicol and acetyltransferase can be measured by FAST CAT chrolamphenicol Acetyltransferase Assay Kit(Wako); and the activity of β-galactosidase can be measured by Aurora Gal-XE (Wako).

(8) If the growth of FM-3-expressing cells in response to Neuromedin U through the activation of MAP kinase can be observed, this growth can be quantified by measuring the activation of MAP kinase, thymidine incorporation, number of cells (e.g. MTT). Using these measurements, the compound that alters the binding between FM-3 and Neuromedin U can be screened.

After adding Neuromedin U or Neuromedin U and a test compound to the cells, and then obtaining MAP kinase fraction from a decomposed cell solution by immunoprecipitation with an anti-MAP kinase antibody, MAP kinase activity can be measured easily by using, for example, MAP Kinase Assay Kit (Wako) and γ-[$^{32}$P]-ATP. For thymidine incorporation activity, Neuromedin U or Neuromedin U and the test compound are added to the inoculated FM-3-expressing cells. Then, [methyl-$^3$H]-thymidine is added thereto. A radioactivity of a labeled thymidine that is taken up into the cells can be measured by dissolving the cells and counting the radioactivity with a liquid scintillation counter.

To measure the growth of FM-3-expressing cells, the cells are inoculated at first, and then Neuromedin U or Neuromedin U and the test compound are added to the cells. Then, MTT (3-(4,5-dimethyl-2-thiazolyl)-2,5-diphenyl-2H-tetrazolium bromide) is added thereto. After dissolving the cells in iso-propanol which acidified by hydrochloric acid, MTT fromazan which is formed from MTT in the cells was measured by absorption at 570nm.

The details of screening method using the labeled thymidine incorporation activity for the compound that alters the binding between FM-3 and Neuromedin U are as follows.

FM-3-expressing cells are placed at $5x10^3$ cell/well on a 24-well plate and cultivated for a day. Then, the cells are cultivated in the medium without serum to make the cells to become starved condition. Neuromedin U or Neuromedin U and a test compound are added to the cells and the cells are cultivated for 24 hours. [methyl-$^3$H] - thymidine at 0.015MBq/well is added thereto and the cells are cultivated for 6 hours. The cells are washed with PBS(-), methanol is added thereto and kept still for 10 minutes. Then, 5% trichloro acetate was added and kept still for 15 minutes. The fixed cells are washed with distilled water 4 times. The cells are dissolved in 0.3 N sodium hydroxide. A radioactivity in the decomposed cell solution is measured with a liquid scintillation counter. An influence of the compound that alters the binding between Neuromedin U and FM-3 can be measured by comparing the increase in the radioactivity in thymidine incorporation with the case with the single administration of Neuromedin U. The compound that inhibits the increase in the radioactivity by Neuromedin U administration can be selected as a candidate substance that is capable of altering the binding property between Neuromedin U and FM-3. On the other hand, by administrating the test compound singly and observing the increase in the radioactivity like that with Neuromedin U, an agonist can be screened.

(9) On adding Neuromedin U to FM-3-expressing cells, K-channel becomes activated, and K ions in the cells flow out of the cells. At this time, Rb ions which belong to the related element, flow out of the cells through K channel as well as K ions. A labeled Rb ([$^{86}$RB]) is added to the cells to make the cells incorporate it. Then, by measuring the efflux of [$^{86}$RB], the activity of Neuromedin U can be measured. The details of screening method for the compound that alters the binding between Neuromedin U and FM-3 by using the efflux activity of [$^{86}$RB] are as follows.

Two days after placing in a 24-well plate, FM-3-expressing cells are kept warm for 2 hours in the medium containing $^{86}$RBCl (1mCi/ml). The cells were washed well to remove $^{86}$RBCl completely from the extracellular solution. Neuromedin U or Neuromedin U and a test compound are added to the cells, and the extracellular solution is collected after 30 minutes. A radioactivity therein is measured by a $\gamma$-counter. An influence of the compound that alters the binding between Neuromedin U and FM-3 can be measured by comparing the increase in the radioactivity by efflux of [$^{86}$RB] with the case of a single administration of Neuromedin U. The compound that inhibits the increase in the radioactivity by administrating Neuromedin U, can be selected as a candidate substance that is capable of altering the binding property between Neuromedin U and FM-3. On the other hand, by administrating the test compound singly and by observing the increase in the radioactivity like that by Neuromedin U, an agonist can be screened.

(10) FM-3-expressing cells changes extracellular pH (acidification rate) in response to Neuromedin U. By measuring such change with a site sensor device (Molecular Device), the activity of Neuromedin U can be measured. The details of screening method for the compound that alters the binding between Neuromedin U and FM-3 by measuring the extracellular pH change with the site sensor device are as follows.

FM-3-expressing cells are cultivated in a capsule of the site sensor over night. The cells are set to the chamber of the device and they are refluxed with RMPI1640 medium supplemented with 0.1% BSA (Molecular Divice) for 2 hours until the extracellular pH become stable. After the pH becomes stable, measured is the pH change of the medium caused by refluxing the medium containing Neuromedin U or Neuromedin U and a test compound on the cells. An influence of the compound that alter the binding of Neuromedin U and FM-3 can be measured by comparing the change of extracellular pH in FM-3-expressing cells with that by the single administration of Neuromedin U. The compound that inhibits the change of extracellular pH by administrating Neuromedin U can be selected as a candidate substance that is capable of altering the binding property between Neuromedin U and FM-3. On the other hand, by administrating the test compound singly and observing the extracellular pH change like that by Neuromedin U, an agonist can be screened.

(11) A sex pheromone receptor STe2 of haploid $\alpha$-mating Type (MAT $\alpha$) of yeast (Saccharomyces cerevisiae) is coupled with G-protein Gpa1. In response to sex pheromone $\alpha$-mating factor, the receptor activates MAP kinase, and sequentially Far1 (cell-cycle arrest) and transcription activation factor Stel2. Ste12 induces the expression of various proteins related to the mating, including FUSI. On the other hand, the regulatory factor Sst2 works in an inhibitory manner in the above process. In this system, yeast into which the receptor gene is introduced is prepared.

The intracellular signal transduction system in the yeast is activated by a receptor agonist stimulation, and an experiment for the measurement system of the reaction between the receptor agonist and the receptor is conducted by using the growth, etc. resulted from the activation of the intracellular signal transduction as an index (Pausch, M.H., Treinds in Biotechnology, vol.15, pp. 487-494 (1997)). Using such system of the receptor gene introduced yeast, the compound that alters the binding between Neuromedin U and FM-3 can be screened.

The genes encoding Ste2 and Gpal of MAT α yeast are removed and the genes encoding FM-3 and Gpa1-Gai2 fused protein are introduced instead. The gene encoding Far is removed to prevent cell cycle arrest and the gene encoding Sst is removed to increase the sensitivity of response to Neuromedin U. Moreover, the FUS1-H1S3 gene in which FUS1 is connected with a histidine biosynthesis gene HIS3 is introduced. The above-mentioned genetic recombinant method can be easily carried out according to, for example, the method reported by Price (Price, L. A. et al., Molecular and Cellular Biology, vol. 15, pp.6188-6195 (1995)), using FM-3 gene in place of a somatostatin receptor type 2 (SSTR2). The transformant of yeast constructed according to the above-mentioned method reacts to Neuromedin U that is a ligand of FM-3 with a high sensitivity, causing the activation of MAP kinase and production of a histidine biosynthetic enzyme so that it can grow in a histidine deficient medium. Using this system, the response of FM-3 expressing yeast to Neuromedin U can be observed by using the growth of yeast in the histidine deficient medium as an index. The screening method for the compound that alters the binding between Neuromedin U and FM-3 is as follows.

The above-prepared transformant of yeast is cultured in a complete synthetic medium liquid overnight, added at a concentration of $2X10^4$ cell/ml to a melted agar from which histidine is removed, and plated on square Petri dish (9x9cm). After the agar becomes hard, a sterilized filter paper absorbing Neuromedin U or Neuromedin U and a test compound, is placed on the surface of agar and the transformant is cultured for 3 days at 30 °C. An influence of the compound that alters the binding of Neuromedin U and FM-3 can be measured by comparing the growth of yeast around the filter paper with the case of singl administration of Neuromedin U. The compound that inhibits the growth of yeast by Neuromedin U administration can be selected as a candidate substance that is capable of altering the binding property of Neuromedin U and FM-3. On the other hand, an agonist can be screened by administrating only the test compound and observing the growth of yeast like the growth observed in Neuromedin U administration. Furthermore, the transformant of yeast is cultured on the agar containing Neuromedin U, and by observing an influence on the growth of yeast over the surface in Petri dish around the filter paper absorbing a test compound, an influence of the compound that alters the binding of Neuromedin U and FM-3 can be measured.

(12) An oocyte of Xenopus Laevis is injected with RNA of FM-3 gene and stimulated by Neuromedin U. As a result, intracellular $Ca^{2+}$ concentration increases and calcium-activated chloride current occurs. This change can be detected as a change of membrane potential (similar to the case where K ion concentration gradient is changed). By observing the reaction caused in the FM-3-introduced Xenopus Laevis oocytes by Neuromedin U, the compound that has an influence on the binding between Neuromedin U and FM-3 can be screened.

A block of oocytes, collected from a female Xenopus Laevis numbed by ice-cooling, was treated with collagenase(0.5mg/ml) dissolved in MBS solution (88mM NaCl, 1mM KCl, 0.41mM $CaCl_2$, 0.33mM $Ca(NO_3)_2$, 0.82mM $MgSO_4$, 2.4mM $NaHCO_3$, 10mM HEPES, pH7.4), shaking at 150rpm for 1-6 hours at 19°C until the block of cells gets loose. After washing for three times with MBS, FM-3 mRNA (50ng/50nl) is microinjected into an oocyte with a micromanipulator. FM-3 mRNA can be prepared from tissues or cells, or by in vitro transcription from a plasmid. The oocyte is cultured in MBS solution for 3 days at 20°C, and placed in a pit of a voltage clamp devise where Ringer solution flows. Glass microelectrodes for voltage clamp and voltmeter are inserted into the cell and the cathode is placed outside of the cell. After the potential become stable, the change in potential is recorded after passing the Ringer solution containing Neuromedin U or Neuromedin U and a test compound. An influence of the compound that alters the binding between Neuromedin U and FM-3 is measured by comparing the membrane potential change of FM-3 introduced Xenopus Laevis oocyte with the case of single administration of Neuromedin U. The compound that inhibits the cell membrane potential change can be selected as a candidate substance that is capable of altering the binding between FM-3 and Neuromedin U. On the other hand, an agonist can be screened by administrating only the test compound and observing the cell membrane potential change like the change observed in Neuromedin U administration.

In this system, poly $(A)^+$ RNA of various G-protein genes can be introduced to amplify the change so that the reaction can be measured easily. Also, the poly $(A)^+$ RNA of protein gene, such as aequorin which radiates light in the presence of Ca ion is injected as well so that the reaction can be measured by observing the radiation of light instead of the membrane potential change.

The screening kit for the compound or a salt thereof that alters the binding property of Neuromedin U with FM-3 comprises FM-3, the cells containing FM-3, or the membrane fraction of the cells containing FM-3; and Neuromedin U.

Examples of the screening kit of the present invention are as follows.

1. Reagents for screening

(1) Buffer for measurement and washing

Hanks' balanced salt solution(Gibco Co.) supplemented with 0.05% bovine serum albumin (Sigma Co.).
The solution is sterilized by filtration through a 0.45 μm filter, and stored at 4°C or may be prepared at use.

(2) Standard FM-3

CHO cells expressing FM-3 which are plated on a 12-well plate at a density of $5 \times 10^5$ cells/well, and cultured at 37°C under 5% $CO_2$ and 95% air for 2 days.

(3) Labeled ligands

Neuromedin U labeled with [$^3$H], [$^{125}$I], [$^{14}$C], [$^{35}$S], etc., which is dissolved in an appropriate buffer, and stored at 4°C or -20°C, and diluted to 1 μM with the measurement buffer at use.

(4) Standard ligand solution

Neuromedin U is dissolved in PBS containing 0.1% bovine serum albumin(Sigma Co.) at a final concentration of 1 mM, and stored at -20°C.

2. Measurement method

(1) Cells expressing FM-3 are cultured in a 12-well culture plate and washed twice with 1 ml of the measurement buffer, and 490 μl of the measurement buffer is added to each well.
(2) After adding 5 μl of $10^{-3}$ - $10^{-10}$ M test compound solution, and then 5 μl of a labeled Neuromedin U, the cells are incubated at room temperature for an hour. To determine the amount of the non-specific binding, 5 μl of $10^{-3}$ M Neuromedin U is added in place of the test compound.
(3) The reaction solution is removed, and the wells are washed 3 times with the washing buffer. The labeled Neuromedin U bound to the cells is dissolved in 0.2N NaOH-1% SDS, and mixed with 4 ml of liquid scintillator A (Wako Pure Chemical Industries, Ltd.)
(4) The radioactivity is measured using a liquid scintillation counter (Beckman Co.), and the percent maximum binding (PMB) is calculated by the equation below.

$$PMB = [(B - NSB)/(B_0 - NSB)] \times 100$$

PMB : Percent maximum binding
B : Value obtained in the presence of a test compound
NSB : Non-specific binding
$B_0$ : Maximum binding

The compound obtained by the screening method or the screening kit of the present invention, or a salt thereof is a compound that alters (inhibits or enhances) the binding between Neuromedin U and FM-3. Concretely, it is a compound having the FM-3-mediated cell stimulating activity(so-called FM-3 agonist) or a compound not having the cell stimulating activity (so-called FM-3 antagonist). The compounds include peptides, proteins, non-peptide compounds, synthetic compounds, and fermentation products. They may be novel or known compounds.
The evaluation method for determining whether it is the FM-3 agonist or FM-3 antagonist mentioned above is described in (i) and (ii) below.
(i) By conducting binding assay according to the screening methods (1) - (3) above, the compound that alters (especially, inhibits) the binding property between Neuromedin U and FM-3 is obtained. Then, the obtained compound is assayed as to whether it has the above-mentioned FM-3-mediated cell stimulating activities or not. The compound having the cell stimulating activities or a salt thereof is determined to be a FM-3 agonist, and the compound not having the cell stimulating activities or a salt thereof is determined to be a FM-3 antagonist.
(a) The above-mentioned FM-3-mediated cell- stimulating activity is measured after contacting a test compound to cells having FM-3. The compound having the cell stimulating activity or salt thereof is a FM-3 agonist. (b) The above-mentioned FM-3-mediated cell- stimulating activity is measured and compared between when the compound that activates FM-3 (e.g. Neuromedin U or FM-3 agonist) is brought in contact with cells having FM-3 and when the compound that activates FM-3 and the test compound are brought in contact with cells having FM-3. The compound or a salt thereof which may reduce the cell stimulating activity induced by the compound that activates FM-3 is a FM-3 antagonist.

Since FM-3 agonists have the same physiological activities as that of Neuromedin U, they are useful as safe and low-toxic pharmaceuticals.

On the other hand, since FM-3 antagonists can inhibit the physiological activities of Neuromedin U, they are useful as safe and low-toxic pharmaceuticals for inhibiting the receptor activities.

Since Neuromedin U or a salt thereof relates to the smooth muscle contraction, the function of increasing blood pressure, the adjustment of ion-transportation in intestine, and the increase in ACTH and subsequent increase in corticosterone after its hypodermic administration, it can be used as prophylactic and/or therapeutic agents for hypotension, local vasoconstriction agents, remedies for diseases associated with dysfunction of pituitary hormonal secretion. Thus, among compounds obtainable by the above-mentioned screening methods or screening kits, FM-3 agonists can be used as prophylactic and/or therapeutic agents for hypotension, local vasoconstriction agents, remedies for diseases associated with dysfunction of pituitary hormonal. secretion, and FM-3 antagonists can be used as prophylactic and/or therapeutic agents for hypertension, myocardial dysfunction, acute renal failure, stress-related diseases (e.g. (1) diseases of cardiovascular system (angina pectoris, myocardial infarction, arrhythmia, etc.), (2) diseases of respiratory system (bronchial asthma, hyperpnea syndrome, etc.), (3) diseases of musculoskeletal system (e.g. chronic arthorheumatism, lumbago, migraine, tension headache, etc.), (4) other diseases (e.g. diabetes, climacteric disorder, chronic pain, decrease of immunity, etc.), diseases of digestive system (gastric ulcer, ulcerative colitis, etc).

Moreover, since Neuromedin U and a salt thereof has the function of controlling appetite, among compounds obtainable by the above-mentioned screening methods or screening kits on the basis of said function, FM-3 agonists are used as anorectic agents, anti-adiposis agents, remedies for bulimia and polyphagia, and FM-3 antagonists are used as aperitive agents.

For the salt of the compound that can be obtained according to the above-mentioned screening method or the screening kit, for example, the pharmacologically acceptable salt is used. The examples are a salt with an inorganic base, a salt with an organic base, a salt with an inorganic acid, a salt with an organic acid, a salt with a basic or acidic amino acid and so on.

The preferred examples of salt with an inorganic base include an alkali metal salt such as sodium salt, potassium salt; alkali earth metal salt, calcium salt and magnesium salt; and aluminum salt, ammonium salt, etc.

The preferred examples of salt with an organic base include trimethylamine salt, triethylamine salt, pyridine salt, picoline salt, 2,6-lutidine salt, ethanolamine salt, diethanolamine salt, triethanolamine salt, cyclohexylamine salt, dicyclohexylamine salt, N,N'-dibenzylethylenediamine salt, etc.

The preferred examples of salt with an inorganic acid are hydrochloric acid salt, hydrobromic acid salt, sulfuric acid salt, phosphoric acid salt, etc.

The preferred examples of salt with an organic acid are formic acid salt, acetic acid salt, propionic acid salt, fumaric acid salt, oxalic acid salt, tartaric acid salt, maleic acid salt, citric acid salt, succinic acid salt, malic acid salt, methanesulfonic acid salt, benzenesulfonic acid salt, benzoic acid salt, etc.

The preferred examples of salt with a basic amino acid include a salt with arginine, lysine, ornithine, etc. The preferred examples of salt with an acidic amino acid include a salt with aspartic acid, glutamic acid, etc.

When the compound obtainable using the screening method or the screening kit or a salt thereof is used as the above-mentioned drug, it can be used as follows.

When the compound obtainable using the screening method or the screening kit or a salt thereof is used as the above-mentioned drug, it can be prepared by publicly known methods. For example, the compound can be used orally as tablets having sugar coating or enteric coating as necessary, capsules, elixirs and microcapsules, or parenterally as an injection, such as an aseptic solution or suspension with water or other pharmaceutically acceptable. For example, these preparations can be produced by admixing physiologically acceptable carriers, flavors, excipients, vehicles, preservatives, stabilizers and binders with the compound or a salt thereof of the present invention in a generally acceptable unit dose required for pharmaceutical formulation. The amount of the active ingredient in these pharmaceutical preparations is designed to have a suitable dose in the designated range.

The additives which can be admixed in the tablets, capsules etc. include, for example, binders such as gelatin, corn starch, tragacanth, gum arabic; excipients such as crystalline cellulose; swelling agents such as corn starch, gelatin and alginic acid; lubricants such as magnesium stearate; sweeteners such as sucrose, lactose and saccharine; and flavors such as peppermint, akamono oil and cherry. When a capsule is in a unit dosage form, liquid carriers such as fats and oils can be contained in the materials described above. The aseptic composition for injection can be formulated according to conventional pharmaceutical formulation by dissolving or suspending the active material and naturally occurring vegetable oils such as sesame oil and coconut oil in vehicles such as injection water.

The aqueous liquid for injection includes, for example, physiological saline or an isotonic solution containing glucose and other supplementary agents (e.g., D-sorbitol, D-mannitol, sodium chloride etc.), and may be used in combination with suitable solubilizer such as alcohols (e.g., ethanol etc.), polyalcohols (e.g., propylene glycol, polyethylene glycol etc.) and nonionic surfactants (e.g., Polysorbate 80™, HCO-50 etc.). The oily liquid includes, for example, sesame oil, soybean oil etc., and may be used in combination with solubilizer such as benzyl benzoate, benzyl alcohol etc.

Further, it may contain buffers (e.g., phosphate buffer, sodium acetate buffer etc.), soothing agents (e.g., benzalkonium chloride, procaine hydrochloride etc.), stabilizers (e.g., human serum albumin, polyethylene glycol etc.), preservatives (e.g., benzyl alcohol, phenol etc.), antioxidants etc. Usually, the prepared injection is filled into suitable ampoules.

The pharmaceutical preparation thus obtained is safe and low toxic so that it can be administered into warm-blooded animals (e.g., human, guinea pig, rat, mouse, pig, sheep, cow, monkey, dog and chicken), amphibian (e.g. frog) and fish.

The dose of the compound or a salt thereof (especially antagonists), which is obtained by the screening method or the screening kit of the present invention, varies depending on conditions. In oral administration, e.g., for the adult patient with hypertension, the dose is normally about 0.1 mg to about 1000 mg, preferably about 1.0 to about 300 mg, and more preferably about 3.0 to about 50 mg per day (as 60 kg body weight). In parenteral administration, the single dose varies depending on subject to be administered, conditions, routes for administration, etc. but it is advantageous, e.g., for the adult patient with hypertension, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The antibody to the above-mentioned rat FM-3 of the present invention, that is, the antibody to the protein or a salt thereof which may contain (1) an amino acid sequence represented by SEQ ID NO:19, (2) an amino acid sequence represented by SEQ ID NO:19, wherein 1 to 30 of amino acids are added, (3) an amino acid sequence represented by SEQ ID NO:19, wherein 1 to 30 of amino acids are substituted by other amino acids, or (4) an amino acid sequence represented by SEQ ID NO:19, wherein 1 to 30 of amino acids are added and 1 to 30 of amino acids are substituted by other amino acids, is described below.

Antibodies to the rat FM-3 of the present invention or a salt thereof may be any of polyclonal or monoclonal antibodies which are capable of recognizing the rat FM-3 of the present invention or a salt thereof.

The antibodies to the rat FM-3 of the present invention or a salt thereof may be manufactured by publicly known methods for manufacturing antibodies or antisera, using as antigens the rat FM-3 of the present invention.

[Preparation of monoclonal antibody]

(a) Preparation of monoclonal antibody-producing cells

The rat type FM-3 of the present invention is administered to warm-blooded animals either solely or together with carriers or diluents to the site where the production of antibody is possible by the administration. In order to potentiate the antibody productivity upon the administration, complete Freund's adjuvant or incomplete Freund's adjuvant may be administered. The administration is usually carried out once every two to six weeks and two to ten times in total. Examples of the applicable warm-blooded animals are monkeys, rabbits, dogs, guinea pigs, mice, rats, sheep and goats, with the use of mice and rats being preferred.

In the preparation of monoclonal antibody-producing cells, warm-blooded animals, e.g. mice are immunized with an antigen, a individual having detectable antibody titer is selected, then spleen or lymph node is collected after two to five days after the final immunization, and antibody-producing cells contained therein are fused with myeloma cells to give monoclonal antibody-producing hybridomas. Measurement of the antibody titer in antisera may be carried out, for example, by reacting a labeled polypeptide with the antiserum, followed by assaying the binding activity of the labeling agent bound to the antibody. The fusion may be carried out, for example, by the known method by Koehler and Milstein (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, AP-1, etc. P3U1 is preferably employed. A preferred ratio of the number of the antibody-producing cells used (spleen cells) to the number of myeloma cells is within a range of approximately 1:1 to 20:1. When PEG (preferably, PEG 1000 to PEG 6000) is added in a concentration of approximately 10 to 80% followed by incubating at 20 to 40°C, preferably at 30 to 37°C for 1 to 10 minutes, an efficient cell fusion can be carried out.

Various methods can be used for screening a monoclonal antibody-producing hybridoma. Examples of such methods include a method which comprises adding the supernatant of hybridoma to a solid phase (e.g., microplate) adsorbed with the rat FM-3 antigen of the present invention directly or together with a carrier, adding an anti-immunoglobulin antibody (where mouse cells are used for the cell fusion, anti-mouse immunoglobulin antibody is used) labeled with a radioactive substance or an enzyme or Protein A, and detecting the monoclonal antibody bound to the solid phase; and a method which comprises adding the supernatant of hybridoma to a solid phase adsorbed with an anti-immunoglobulin antibody or Protein A, adding the rat FM-3 labeled with a radioactive substance or an enzyme, and detecting the monoclonal antibody bound to the solid phase.

The monoclonal antibody can be selected according to publicly known methods or their modifications. In general, the selection can be effected in a medium for animal cell supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used. The cultivation is carried out generally at 20°C to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out in the same way as the case of a polyclonal antibody, by conventional methods for separation and purification of immunoglobulins (for example, salting-out, alcohol precipitation, isoelectric point precipitation, electrophoresis, adsorption and desorption with ion exchangers (e.g. DEAE), ultracentrifugation, gel filtration, or a affinity purification method which comprises collecting only an antibody with an activated adsorbent such as an antigen-binding solid phase, Protein A or Protein G, and dissociating the binding to obtain the antibody.

[Preparation of polyclonal antibody]

The polyclonal antibody to the rat FM-3 of the present invention can be manufactured by publicly known methods or modifications thereof. For example, a warm-blooded animal is immunized with the complex of the immunogen(the receptor protein) and a carrier protein in a manner similar to the method described above for the production of monoclonal antibodies. The fraction containing the antibody to the rat FM-3 of the present invention is collected from the immunized animal to carry out separation and purification of the antibody.

In the complex of immunogen and carrier protein used to immunize a warm-blooded animal, any type of carrier protein may be crosslinked to the hapten in any mixing ratio of carrier to hapten, as long as the antibody is efficiently produced to the immunized complex. For example, bovine serum albumin, bovine thyroglobulin or keyhole limpet hemocyanin, etc. is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every 2 to 6 weeks and 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins as performed in the separation and purification of monoclonal antibodies described above.

The antibodies to the rat FM-3 of the present invention are capable of recognizing specifically the rat FM-3 of the present invention. Therefore, the antibodies can be used to quantify the protein of the present invention in a test fluid, especially for quantification by the sandwich immunoassay. Thus, the present invention provides, for example, the following quantification methods:

(i) a method of quantifying the rat FM-3 of the present invention in a test fluid, which comprises reacting the antibody to the rat FM-3 of the present invention competitively with the test fluid and the labeled rat FM-3; and measuring the ratio of the labeled rat FM-3 bound to the antibody; and,

(ii) a method of quantifying the rat FM-3 of the present invention in a test fluid, which comprises reacting the test fluid with the antibody to the rat FM-3 of the present invention immobilized on a carrier and the labeled antibody to the rat FM-3 of the present invention simultaneously or sequentially; and measuring the activity of the label on the immobilizing carrier.

In (ii) described above, it is preferred that one antibody recognizes the N-terminal region of the rat FM-3 of the present invention, and another antibody reacts with the C-terminal region of the rat FM-3 of the present invention.
Using monoclonal antibodies to the rat type FM-3 of the present invention (hereinafter sometimes referred to as the monoclonal antibodies of the present invention), the rat type FM-3 of the present invention can be assayed and also detected by tissue staining or the like. For this purpose, an antibody molecule itself may be used, or $F(ab')_2$, Fab' or Fab fractions of the antibody molecule may also be used. Assay methods using antibodies to the rat type FM-3 of the present invention are not particularly limited. Any assay method can be used, so long as the amount of antibody, antigen, or antibody-antigen complex corresponding to the amount of antigen (e.g., the amount of the rat FM-3) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.
As the labeling agent for the methods using labeled substances, there are employed, for example, radioisotopes, enzymes, fluorescent substances, luminescent substances, etc. For the radioisotope, for example, [$^{125}I$], [$^{131}I$], [$^{3}H$] and [$^{14}C$] are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used are fluorescamine and fluorescein isothiocyanate. For the luminescent substance, for example, luminol, luminol derivatives, luciferin, and lucigenin. Furthermore, the biotin-avidin system may be used for binding antibody or antigen to the label.
For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like are used.
In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the rat FM-3 of the present invention in the test fluid can be quantified. The order of the primary and secondary reactions may be reversed, and the reactions may be performed simultaneously or with an interval. The used labeling agent and method for immobilization may be the same as those described above.
In the immunoassay by the sandwich method, the antibody used for immobilized or labeled antibodies is not necessarily one species, but a mixture of two or more species of antibody may be used to increase the measurement sensitivity.
In the methods of assaying the receptor protein by the sandwich method of the present invention, antibodies that bind to different sites of rat FM-3 are preferably used as the monoclonal antibodies of the present invention for the primary and secondary reactions. That is, in the antibodies used for the primary and secondary reactions are, for example, when the antibody used in the secondary reaction recognizes the C-terminal region of the rat FM-3, it is preferable to use the antibody recognizing the region other than the C-terminal region for the primary reaction, e.g., the antibody recognizing the N-terminal region.
The monoclonal antibodies of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using an antibody in a soluble form, polyethylene glycol for B/F separation and a secondary antibody to the soluble antibody; and an solid phase method either using the immobilized primary antibody, or using the soluble primary antibody and the immobilized secondary antibody.
In the immunometric method, after antigen in a test fluid and immobilized antigen are competitively reacted with a definite amount of labeled antibody, the solid phase is separated from the liquid phase. Alternatively, after antigen in a test fluid and an excess amount of labeled antibody are reacted, and then immobilized antigen is added to adsorb the unreacted labeled antibody, the solid phase is separated from the liquid phase. Then, the amount of the label in either phase is measured to quantify the antigen in the test fluid.
In the nephrometry, an insoluble precipitate produced after the antigen-antibody reaction in gel or solution is quantified. When the amount of antigen in the test fluid is so small that only a small amount of precipitate is obtained, laser nephrometry using scattering of laser is advantageously employed.

For applying these immunological methods to the measuring methods of the present invention, any particular conditions or procedures are not required. Systems for measuring the rat FM-3 of the present invention or its salts are constructed by adding the usual technical consideration in the art to the conventional conditions and procedures. For the details of these general technical means, reference can be made to the following reviews and texts. See, for example, Hiroshi Irie, ed. "Radioimmunoassay" (Kodansha, published in 1974); Hiroshi Irie, ed. "Sequel to the Radioimmunoassay" (Kodansha, published in 1979); Eiji Ishikawa, et al. ed. "Enzyme immonoassay" (Igakushoin, published in 1978); Eiji Ishikawa, et al. ed. "Immunoenzyme assay" (2nd ed.) (Igakushoin, published in 1982); Eiji Ishikawa, et al. ed. "Immunoenzyme assay" (3rd ed.) (Igakushoin, published in 1987); Methods in ENZYMOLOGY, Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies))(all published by Academic Press Publishing).

The antibodies to the rat FM-3 of the present invention can also be used for detecting specifically the rat FM-3 of the present invention present in test samples such as body fluids or tissues. The antibodies may also be used for preparation of antibody columns for purification of the rat FM-3 of the present invention, for detection of the rat FM-3 of the present invention in each fraction upon purification, and for analysis of the behavior of the rat type FM-3 of the present invention in the test cells.

In the specification and drawings, the codes of bases and amino acids are denoted in accordance with the IUPAC-IUB Commission on Biochemical Nomenclature or the conventional codes in the art, examples of which are shown below. For amino acids that have the optical isomer, L form is represented unless otherwise indicated.

| | |
|---|---|
| DNA : | deoxyribonucleic acid |
| cDNA : | complementary deoxyribonucleic acid |
| A : | adenine |
| T : | thymine |
| G : | guanine |
| C : | cytosine |
| Y : | thymine or cytosine |
| N : | adenine, guanine, cytosine or thymine |
| R : | adenine or guanine |
| M : | adenine or cytosine |
| W : | adenine or thymine |
| S : | guanine or cytosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecyl sulfate |
| TFA : | trifluoroacetic acid |
| EIA : | enzyme immunoassay |
| Gly or G: | glycine |
| Ala or A: | alanine |
| Val or V: | valine |
| Leu or L: | leucine |
| Ile or I: | isoleucine |
| Ser or S: | serine |
| Thr or T: | threonine |
| Cys or C: | cysteine |
| Met or M: | methionine |
| Glu or E: | glutamic acid |
| Asp or D: | aspartic acid |
| Lys or K: | lysine |

Arg or R: arginine
His or H: histidine
Phe or F: phenylalanine
Tyr or Y: tyrosine
Trp or W: tryptophan
Pro or P : proline
Asn or N: asparagine
Gln or Q: glutamine
pGlu : pyroglutamic acid
Me : methyl
Et : ethyl
Bu : butyl
Ph : phenyl
TC : thiazolidine-4(R)-carboxamide
Bom : benzyloxymethyl
NMP : N-methylpyrrolidone
PAM : phenylacetoamidemethyl

The substituents, protective groups and reagents, which are frequently used throughout the specification, are shown by the following abbreviations.

Tos : p-toluenesulfonyl
HONB : 1-hydroxy-5-norbornene-2,3-dicarboximide
Bz1 : benzyl
Z benzyloxycarbonyl
Br-Z : 2-bromobenzyloxycarbonyl
Cl-Z : 2-chlorobenzyloxycarbonyl
Boc : t-butoxycarbonyl
Host : 1-hydroxybenztriazole
DCC : N,N'-dicyclohexylcarbodiimide
TFA : trifluoroacetic acid
Fmoc : N-9-fluorenylmethoxycarbonyl
DNP: dinitrophenyl
Bum : t-butoxymethyl
Trt : trityl
BSA : bovine serum albumin
CHAPS : 3-[(3-colamidepropyl)dimethylanmmonio]-1-propane sulfonate
PMSF : phenylmethylsulfonylfluoride
E64 : (L-3-trans-caroboxoirane-2-carbonyl) L-leucyl-agumatin
GDP : Guanosine-5'-diphosphate
MEM $\alpha$: minimum essential medium alpha
Fura-2AM: 1-[6-amino-2-(5-carboxy-2-oxazolyl)-5-benzofuranyloxy]-2-(2-amino-5 methylphenoxy)-ethane-N', N', N', N'-tetra acetic acid-pentacetoxymethyl ester
HBSS: Hanks' Balanced Salt Solution
Fluo-3AM: 1-[2-amino-5-(2,7-dichloro-6-hydroxy-3-oxy-9-xanthenyl)phenoxy]-2-(2-amino-5-methylphenoxy) ethane-N',N',N',N'-tetra acetic acid pentaacetoxymethyl ester
HEPES: 2-[4-(2-hydroxyethyl)-1-piperazinyl] ethanesulfonic acid
MeBzl: 4-methylbenzyl
NMP : N-methylpyrrolidone

The sequence identification numbers in the sequence listing of the specification indicates the following sequence, respectively.

[SEQ ID NO:1]
This shows the amino acid sequence of human FM-3.
[SEQ ID NO:2]
This shows the nucleic acid sequence of DNA encoding human FM-3 which has the amino acid sequence shown by SEQ ID NO:1.

[SEQ ID NO:3]
This shows the amino acid sequence of mouse FM-3.
[SEQ ID NO:4]
This shows the nucleic acid sequence of DNA encoding mouse FM-3 which has the amino acid sequence shown by SEQ ID NO:3.
[SEQ ID NO:5]
This shows the amino acid sequence of pig Neuromedin U-8.
[SEQ ID NO:6]
This shows the amino acid sequence of dog Neuromedin U-8.
[SEQ ID NO:7]
This shows the amino acid sequence of chicken Neuromedin U-9.
[SEQ ID NO:8]
This shows the amino acid sequence of guinea pig Neuromedin U-9.
[SEQ ID NO:9]
This shows the amino acid sequence of rat Neuromedin U-23.
[SEQ ID NO:10]
This shows the amino acid sequence of frog Neuromedin U-23.
[SEQ ID NO:11]
This shows the amino acid sequence of human Neuromedin U-25.
[SEQ ID NO:12]
This shows the amino acid sequence of pig Neuromedin U-25.
[SEQ ID NO:13]
This shows the amino acid sequence of dog Neuromedin U-25.
[SEQ ID NO:14]
This shows the amino acid sequence of chicken Neuromedin U-25.
[SEQ ID NO:15]
This shows the amino acid sequence of frog Neuromedin U-25.
[SEQ ID NO:16]
This shows an amino acid sequence of a partial peptide of Neuromedin U-25. This corresponds to an amino acid sequence at the 4 to 8 positions in the amino acid sequence represented by SEQ ID NO: 5.
[SEQ ID NO:17]
This shows the nucleic acid sequence of FM3F2 used in Example 1.
[SEQ ID NO:18]
This shows the nucleic acid sequence of FM3R2 used in Example 1.
[SEQ ID NO:19]
This shows the amino acid sequence of rat FM-3 obtained in Example 3.
[SEQ ID NO:20]
This shows the nucleic acid sequence of DNA encoding rat FM-3 which has the amino acid sequence shown by SEQ ID NO:19.
[SEQ ID NO:21]
This shows the nucleic acid sequence of the primer rFMF described in Example 3.
[SEQ ID NO:22]
This shows the nucleic acid sequence of the primer rFMR described in Example 3.

Escherichia coli transformant JM109/pTArFM3-1, which has DNA encoding the amino acid sequence of rat FM-3 obtained in Example 3 described below was on deposit with the Ministry of International Trade and Industry, Agency of Industrial Science and Technology, National Institute of Bioscience and Human Technology (NIBH), located at 1-1-3, Higashi, Tsukuba-shi, Ibaraki, Japan, as the Accession Number FERM BP-7358 on November 9, 2000 and with Institute for Fermentation (IFO), located at 2-17-85, Juso Honcho, Yodogawa-ku, Osaka-shi, Osaka, Japan, as the Accession Number IFO 16491 on October 24, 2000.

EXAMPLES

The present invention is described in detail below with Examples, but not intended to limit the scope of the present invention thereto.

Example 1 :

Construction of human FM-3-expressing CHO cells

Human FM-3 cDNA was obtained as follows. Two kinds of synthetic DNAs described below were synthesized according to the sequence of human FM-3 reported in Genomics, 52, 223-229 (1998).

FM3F2: 5'-GTCGACCATGGCTTGCAATGGCAGTGCGGCCAGG-3' (SEQ ID NO:17)

FM3R2: 5'-GCTAGCTCAGGATGGATCGGTCTCTTGCTG-3' (SEQ ID NO:18)

Using these synthetic DNAs, the human FM-3 cDNA was obtained from human fetal brain cDNA by PCR. The reaction solution for PCR was composed of 1µl of rat hypothalamus cDNA solution (0.2 ng poly $(A)^+$RNA-derived), 1µl FM3F2 (10µM), 1µl FM3R2 (10µM), 5µl of 10x attached reaction solution, 5µl dNTP (10mM), 1 µl Ex Taq (Takara) and distilled water, which were mixed together to make the total volume 50 µl. The PCR was carried out using Thermal Cycler 9600, started with 95° C for 2 minutes for denaturing, followed by 28 cycles of (1)98° C for 10 seconds, (2)65° C for 10 seconds, (3) 72° C for 90 seconds. Using an aliquot of the PCR product, the amplified 1.2 kb PCR product was confirmed by electrophoresis. Using TA cloning kit (Invitrogen Inc.), the PCR product was subcloned in *Escherichia coli.* From the Escherichia coli obtained by subcloning, the plasmid was extracted using plasmid extracting machine (Kurabo Co., Ltd.), and the nucleic acid sequence of the insert fragment was determined to confirm that the nucleic acid sequence was identical to that of human FM-3 cDNA (SEQ ID NO:2) reported in the above reference. After digesting the plasmid by Sal I and Nhe I, the fragment of 1.2kb human FM-3 cDNA was obtained. Moreover, pAKKO-111H, the expression vector for animal cells, was digested at the multi-cloning site by Sal I and Nhe I and then electophoresed to recover the vector portion. The resulting human FM-3 cDNA fragment and the expression vector were ligated, and then, Escherichia coli JM109 was transformed to obtain E.coli JM109/pAKKOFM3.

Transformant E.coli JM109/pAKKOFM3 was cultured to prepare a large amount of plasmid pAKKOFM3 DNA.

20 µg of the plasmid DNA prepared was dissolved in 1ml of physiological buffered saline (PBS), transferred into the "gene transfer vial" (Wako Pure Chemical Industries, Ltd.), and shaken vigorously with a vortex mixer to form a liposome containing the DNA. $CHO/dhfr^-$ cells of 1 to 2 x $10^6$ were inoculated on Petri's dish having a diameter of 35 mm. After 20 hours of cultivation, the medium was exchanged to the new medium. The liposome solution (25µl) having 0.5µg of the DNA was added to each of the Petri's dishes and incubated for 16 hours to introduce the plasmid DNA to the cells. Then, the medium was exchanged to the new medium and the cells were cultured for one day. Furthermore, the medium was exchanged to a selection medium and said cells were cultured for 3 days. Finally, said cells were treated with trypsin digestion for dissociation. The dissociated cells was inoculated at a low density in the selection medium (minimum essential medium without deoxyribonucleosides and ribonucleosides, alpha medium with 10% dialyzed fetus bovine serum) to select the transformant. Since only the transformants were able to survive in the medium, FM-3-expressing CHO cell lines were established through repeated subcloning selection in the medium.

EXAMPLE 2 :

The cell-stimulating activity of Neuromedin U-25, which is specific to FM-3-expressing cells

FM-3-expressing CHO cells and mock CHO cells, which were cultured in a cell-cultured container, were digested with trypsin and dissociated. The numbers of cells are counted. Then, the cells were inoculated in the capsules for site sensor (2.7 x $10^5$ cells/capsule). After incubating them overnight, the capsules containing the cells were transferred to the sensor chamber for measurement, and then mounted to the workstation of the site sensor. The portion containing the cells was refluxed with low-buffered RMPI medium supplemented with 0.1% bovine serum albumin, and it was left still until the acidification rate became stable. The measurement of the acidification rate was made during pumping cycle of ON for 80 seconds and OFF for 40 seconds(2 minutes to one cycle). A rate of change in extracellular pH from 8 to 30 seconds after the pumping off was calculated as an acidification rate with the site sensor.

Pig Neuromedin-8 (BACHEM, H-5505, SEQ ID NO:5) was dissolved in distilled water at the concentration of 10mM, and then the gradually diluted solutions were prepared with low buffered RPMI medium containing 0.1% BSA.

The dilution was set in one of the two flow paths of the site sensor. The flow path was switched via a valve to expose the Neuromedin U-8 solution to the cells for 7 minutes and 2 seconds. To normalize the differences of the detection sensitivity in each site sensor chamber, the values obtained during 3 cycles immediately before the exposure of the dilution to the cells were set as 100 % for the calculation of the acidification rate on the site sensor soft ware, and the cell reactivity was compared after such a normalization (Fig.1). As a result, the cell-stimulating activity was detected in a does-dependent manner and specific to FM-3-expressing CHO cells.

EXAMPLE 3:

Obtaining Rat FM-3 gene

To obtain Rat FM-3 gene, the following synthetic DNAs were synthesized referring to the primers represented by SEQ ID NO:17 and SEQ ID NO:18 which were used to obtain Human FM-3 gene.

rFMF:5'-GTCGACCATGCTCTCCCCAAATGCTTCAACGGG-3' (SEQ ID NO:21)

rFMR:5'-GCTAGCTTATTCAGGAGGGTCTGTCTCTTGCTC-3' SEQ ID NO:22)

Using the synthetic DNAs, the rat FM-3 gene was obtained from rat brain cDNA by PCR.

The reaction solution for PCR was composed of 1µl of cDNA solution (0.1ng poly (A)$^+$ RNA-derived), 0.5µl rFMF (10µM), 0.5µl rFMR (10µM), 2.5µl of attached 10x reaction solution, 2.5µl dNTP (10mM), 0.5µl Klen Taq DNA polymerase (Clonetech) and 17.5µl of distilled water, which were mixed together to make the total volume 25µl. The PCR was carried out using Thermal Cycler 9600, started with 95° C for 2 minutes for denaturing, followed by 33 cycles of (1)98° C for 10 seconds, (2)65° C for 10 seconds, (3) 72° C for 60 seconds. Using an aliquot of the PCR product, the amplified 1.3 kb PCR product was confirmed by electrophoresis. The sequence of PCR product was directly determined to have the sequence represented by SEQ ID NO:20. The amino acid sequence deduced from the DNA sequence represented by SEQ ID NO:20 was represented by SEQ ID NO:19 and thus the DNA was confirmed to be the Rat FM-3 gene.

INDUSTRIAL APPLICABILITY

The screening method for a compound or a salt thereof that alters the binding property between Neuromedin U and FM-3, characterized by using Neuromedin U and FM-3, can be applied for screening an agent for the treatment/ prevention of hypertension and stress-related diseases. FM-3 antagonist can be used as an agent for the treatment/ prevention of hypertension and stress-related diseases, etc.

**Claims**

1. A method of screening a compound or a salt thereof that alters the binding property between Neuromedin U or a salt thereof and FM-3 or a salt thereof, which is **characterized by** using Neuromedin U, a derivative thereof or a salt thereof and FM-3 or a salt thereof.

2. A kit for screening a compound or a salt thereof that alters the binding property between Neuromedin U or a salt thereof and FM-3 or a salt thereof, which is **characterized by** comprising Neuromedin U, a derivative thereof or a salt thereof and FM-3 or a salt thereof.

3. A compound or a salt thereof that alters the binding property between Neuromedin U or a salt thereof and FM-3 or a salt thereof, which is obtainable using the screening method according to claim 1 or the screening kit according to claim 2.

4. A pharmaceutical composition comprising a compound or a salt thereof according to claim 3.

5. A pharmaceutical composition according to claim 4, which is a therapeutic and/or prophylactic agent for hypertension or stress-related diseases.

6. An agent for controlling appetite, comprising a compound or a salt thereof according to claim 3.

7. A screening method according to claim 1 or a screening kit according to claim 2, where said Neuromedin U is a peptide comprising the same or the substantially same amino acid sequence as that represented by SEQ ID NO:11.

8. A screening method according to claim 1 or a screening kit according to claim 2, where said FM-3 is a protein comprising the same or the substantially same amino acid sequence as that represented by SEQ ID NO:1.

9. A protein or a salt thereof, comprising (i) the amino acid sequence represented by SEQ ID NO:19, (ii) the amino acid sequence represented by SEQ ID NO:19, to which 1 to 30 amino acids are added, (iii) the amino acid sequence represented by SEQ ID NO:19, in which 1 to 30 amino acids are substituted with other amino acids, or (iv) the amino acid sequence represented by SEQ ID NO:19, to which 1 to 30 amino acids are added and in which 1 to 30 amino acids are substituted with other amino acids.

10. A DNA containing a DNA encoding the protein according to claim 9.

11. A DNA according to claim 10 having the nucleic acid sequence represented by SEQ ID NO:20.

12. A recombinant vector containing the DNA according to claim 10.

13. A transformant transformed with the recombinant vector according to claim 12.

14. A method for manufacturing a protein or a salt thereof according to claim 9, **characterized by** cultivating a transformant according to claim 13, and making the transformant produce the protein according to claim 9.

15. An antibody to a protein or a salt thereof according to claim 9.

Fig.1

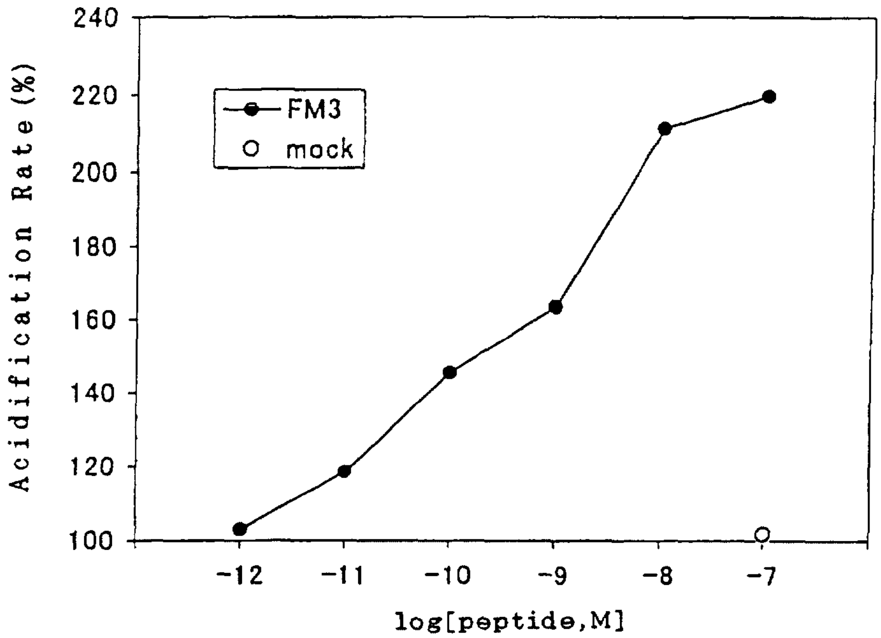
Acidification Rate (%)
240
220
200
180
160
140
120
100
FM3
mock
-12
-11
-10
-9
-8
-7
log[peptide,M]

## INTERNATIONAL SEARCH REPORT

International application No.
PCT/JP00/08363

A. CLASSIFICATION OF SUBJECT MATTER
Int.Cl[7] G01N33/50, 33/15, A61K45/00

According to International Patent Classification (IPC) or to both national classification and IPC

B. FIELDS SEARCHED

Minimum documentation searched (classification system followed by classification symbols)
Int.Cl[7] G01N33/50, 33/15, A61K45/00

Documentation searched other than minimum documentation to the extent that such documents are included in the fields searched

Jitsuyo Shinan Koho 1922-1996 Toroku Jitsuyo Shinan Koho 1994-2001
Kokai Jitsuyo Shinan Koho 1971-2001 Jitsuyo Shinan Toroku Koho 1996-2001

Electronic data base consulted during the international search (name of data base and, where practicable, search terms used)
BIOSIS

C. DOCUMENTS CONSIDERED TO BE RELEVANT

| Category* | Citation of document, with indication, where appropriate, of the relevant passages | Relevant to claim No. |
|---|---|---|
| PX | RYO FUJII, "IDENTIFICATION OF NEUROMEDIN U AS THE COGNATE LIGAND OF THE ORPHAN G PROTEIN-COUPLED RECEPTOR FM-3" THE JOURNAL OF BIOLOGICAL CHEMISTRY, VOL.275, NO.28, pp.21068-21074 (2000) | 1,2,7,8 |
| PX | MASAKI HOSOYA, "IDENTIFICATION AND FUNCTIONAL CHARACTERIZATION OF A NOVEL SUBTYPE OF NUROMEDIN U RECEPTOR" THE JOURNAL OF BIOLOGICAL CHEMISTRY, VOL.275, NO.88, pp.29528-29532 (2000) | 1,2,7,8 |
| PY | "IDENTIFICATION OF RECEPTORS FOR NEUROMEDIN U AND ITS ROLE IN FEEDING", NATURE, VOL.406, NO.6791, pp.70-74 (2000) | 1,2,7,8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 February, 2001 (09.02.01) | 20 February, 2001 (20.02.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/08363

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 3~6
because they relate to parts of the international application that do not comply with the prescribed requirements to such an extent that no meaningful international search can be carried out, specifically:
It is unclear what compounds are particularly included in claims 3 to 6.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

(1) Claims 1 to 8 relate to screening methods and drugs.
(2) Claims 9 to 15 relate to a protein, a DNA and an antibody.
These two groups (1) and (2) of inventions are not considered as relating to a group of inventions so linked as to form a single general inventive concept.

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)